# EUROPÄISCHE PATENTSCHRIFT

(11) **EP 4 646 101 B1**
(45) Veröffentlichungstag und Bekanntmachung des Hinweises auf die Patenterteilung: **02.12.2026**
(21) Anmeldenummer: 23818450.1
(22) Anmeldetag: 06.12.2023
(51) Int. Cl.: A01K 67/368, A01K 67/364

(54) **TEMPERIERTE INSEKTENZUCHTVORRICHTUNG MIT BEFEUCHTUNGSEINRICHTUNG UND VERFAHREN ZUR ANZUCHT VON INSEKTEN**
TEMPERATURE-CONTROLLED INSECTS BREEDING DEVICE WITH HUMIDIFYING DEVICE AND METHOD FOR BREEDING INSECTS
DISPOSITIF DE CULTURE D'INSECTES TEMPÉRÉ AVEC HUMIDIFICATEUR ET PROCÉDÉ DE CULTURE D'INSECTES

(30) Priorität: 02.01.2023 DE 102023200006
(43) Veröffentlichungstag der Anmeldung: 12.11.2025
(73) Patentinhaber: KEPREA, 71000 Mâcon (FR)
(72) Erfinder: BERTSCHI, Patrik, 8953 Dietikon (CH); BERTSCHI, Christoph, 8953 Dietikon (CH)
(74) Vertreter: Santarelli
(86) Internationale Anmeldenummer: PCT/EP2023/084454
(87) Internationale Veröffentlichungsnummer: WO 2024/146734

(56) Entgegenhaltungen:
- WO-A1-2021/148894
- CN-A- 110 476 895
- CN-A- 110 999 874

## Beschreibung

Die Erfindung bezieht sich auf eine Zuchtvorrichtung zur Anzucht von Insekten sowie ein Verfahren für ein Züchten von Insekten. Genauer gesagt bezieht sich die Erfindung auf die Anzucht von Larven der Insekten.

Insekten durchlaufen verschiedene Stadien in ihrem Lebenszyklus: Adulte Tiere legen Eier, aus denen Insektenlarven schlüpfen. Insektenlarven wachsen heran. Aus herangewachsenen Insektenlarven entstehen adulte Tiere.

Beispiele für Insektenlarven sind die Larven des Mehlkäfers, auch Mehlwürmer genannt, sowie Larven der schwarzen Soldatenfliege.

Geschlüpfte Mehlwürmer wachsen über etwa acht Wochen zu einer Größe von etwa 2 cm heran. Die herangewachsenen Mehlwürmer werden vor ihrem Verpuppen geerntet und beispielsweise als Tierfutter oder als Lebensmittel für den menschliche Konsum verwertet.

CN 110 476 895 A und CN 110 999 874 A offenbaren gattungsgemäße Zuchtvorrichtungen für eine Aufzucht von Insektenlarven.

Es ist Aufgabe der Erfindung, Insekten anzuzüchten.

Die Aufgabe wird durch eine Vorrichtung mit den Merkmalen des ersten Anspruchs sowie durch ein Verfahren mit den Merkmalen des Nebenanspruchs gelöst. Die abhängigen Ansprüche betreffen vorteilhafte Ausgestaltungen.

Zur Lösung der Aufgabe dient eine Zuchtvorrichtung mit einem Zuchtbehälter. Die Zuchtvorrichtung ist so eingerichtet, dass Insekten angezüchtet werden können. In den Zuchtbehälter kann ein Substrat gebracht werden, welches frisch geschlüpfte Larven von Insekten und Futter für die Insektenlarven umfasst. Die Larven ernähren sich von dem Futter und wachsen so heran. Haben die Larven eine gewünschte Größe erreicht, so können die Larven geerntet und als Tierfutter verwendet werden.

Die Zuchtvorrichtung umfasst eine Befeuchtungseinrichtung, die so eingerichtet ist, dass das Substrat befeuchtet werden kann. Zusammen mit dem Futter können die Larven dann Feuchtigkeit aufnehmen, die die Larven für ihr Wachstum benötigen.

Die Zuchtvorrichtung umfasst eine Entnahmeeinrichtung. Durch die Entnahmeeinrichtung kann Substrat aus dem Zuchtbehälter entnommen werden. Die Entnahmeeinrichtung kann so eingerichtet sein, dass diese automatisiert Substrat aus dem Zuchtbehälter entnehmen kann. Durch das Entnehmen von Substrat kann der Zuchtbehälter geleert werden, um Larven, die eine gewünschte Größe erreicht haben, ernten zu können. Dies kann beispielsweise 2 bis 10 Wochen nach Beginn der Anzucht der Fall sein.

Die Zuchtvorrichtung kann eine Rücktransporteinrichtung für entnommenes Substrat umfassen. Durch die Rücktransporteinrichtung kann Substrat, das dem Zuchtbehälter durch die Entnahmeeinrichtung entnommen wurde, zurück in den Zuchtbehälter transportiert werden und zwar insbesondere automatisiert. Durch die Entnahme und den Rücktransport kann eine übermäßig hohe Verdichtung des Substrats vermieden werden. Eine übermäßig hohe Verdichtung kann Larven beschädigen oder sogar vollkommen töten.

Die Entnahmeeinrichtung und die Rücktransporteinrichtung können so eingerichtet sein, dass diese innerhalb von 12 Stunden bis fünf Tagen Substrat einmal und zwar nur einmal vollständig aus einem Zuchtbehälter entnehmen und wieder in den Zuchtbehälter zurückgeben. Ist beispielsweise innerhalb von 12 Stunden das Substrat vollständig einmal entnommen und zurückgegeben worden, so kann dies bis zum Ende der Anzucht fortgesetzt werden. Es kann also nach Ablauf der 12 Stunden erneut innerhalb von beispielsweise 12 Stunden vollständig entnommen und zurückgegeben werden.

Die Befeuchtungseinrichtung ist so eingerichtet, dass die Befeuchtungseinrichtung Substrat nach der Entnahme befeuchten kann und zwar vorzugsweise während des Rücktransports. Die Befeuchtungseinrichtung kann in die Rücktransporteinrichtung integriert sein. Es kann ein gewünschter Feuchtigkeitsgrad so auch während der Aufzucht gesteuert und/oder geändert werden. Die Aufzucht kann so verbessert optimiert werden.

Die Zuchtvorrichtung kann eine Verdunstungseinrichtung für ein Kühlen umfassen, mit der im Substrat enthaltene Flüssigkeit gesteuert verdunstet werden kann, wenn sich das Substrat im Zuchtbehälter befindet. Die Verdunstungseinrichtung ist so eingerichtet, dass durch das Verdunsten das Substrat gekühlt wird, also eine Temperatur gehalten oder abgesenkt werden kann. Die Verdunstungseinrichtung kann so eingerichtet sein, dass verdunstete Flüssigkeit aus dem Zuchtbehälter herausgeleitet werden kann. Die Verdunstungseinrichtung kann so eingerichtet sein, dass der Grad der Verdunstung und damit der Grad der Kühlung gesteuert werden können. Eine Verdunstung mittels Ultraschall ist möglich. Die Verdunstungseinrichtung umfasst dann eine Ultraschallquelle. Eine Verdunstung durch einen Luftstrom ist möglich. Die Verdunstungseinrichtung umfasst dann eine Einrichtung für eine Erzeugung eines Luftstroms, der durch den Zuchtbehälter hindurchgeleitet wird. Durch die Verdunstungseinrichtung kann vermieden werden, dass Insektenlarven das Substrat übermäßig aufheizen und so den Anzuchterfolg gefährden.

Insbesondere in Kombination mit der Befeuchtungseinrichtung, der Entnahmeeinrichtung und der Rücktransporteinrichtung kann durch die Verdunstungseinrichtung das Substrat gekühlt werden. Die Aufzucht kann so verbessert optimiert werden.

Die Zuchtvorrichtung kann mehrere Zuchtbehälter umfassen, um Insektenlarven parallel anzüchten zu können. Das Alter von Insektenlarven in einem ersten Zuchtbehälter kann sich von dem Alter von Insektenlarven in einem zweiten Zuchtbehälter unterscheiden. Das Alter von Insektenlarven in einem dritten Zuchtbehälter kann sich von dem Alter der Insektenlarven aus dem ersten und dem zweiten Zuchtbehälter unterscheiden. Es können so zu unterschiedlichen Zeiten Insektenlarven geerntet werden. Eine quasikontinuierliche Produktion von Insektenlarven ist so möglich.

Für jeden Zuchtbehälter kann eine eigene Entnahmeeinrichtung vorhanden sein. Für jeden Zuchtbehälter kann eine eigene Verdunstungseinrichtung zumindest teilweise vorhanden sein. Es kann also auch ein Teil wie zum Beispiel ein Gebläse vorhanden sein, mit dem ein Luftstrom erzeugt wird, der aufgeteilt und in mehrere Zuchtbehälter eingeleitet wird. Es ist zwar auch möglich, dass für jeden Zuchtbehälter eine eigene Befeuchtungseinrichtung und/oder eine eigene Rücktransporteinrichtung vorhanden ist. Es ist jedoch zu bevorzugen, dass Teile einer Befeuchtungseinrichtung und/oder Teile einer Rücktransporteinrichtung gemeinsam von allen Zuchtbehältern genutzt werden, um den technischen Aufwand geringzuhalten. Zumindest teilweise kann also beispielsweise eine Rücktransporteinrichtung für sämtliche Zuchtbehälter vorhanden sein, um Substrat in Zuchtbehälter zurück zu transportieren. Es kann so der technische Aufwand geringgehalten werden.

Die Zuchtbehälter können nebeneinander und/oder hintereinander angeordnet sein. Die Vorrichtung kann beispielsweise 4 bis 20 Zuchtbehälter umfassen. Zuchtbehälter können um Teile einer Rücktransporteinrichtung herum angeordnet sein, um den technischen Aufwand für einen Rücktransport geringhalten zu können. Zuchtbehälter können beispielsweise um ein Becherwerk als Teil der Rücktransporteinrichtung herum gruppiert sein.

Ein Zuchtbehälter kann eine nach oben offene Eintrittsöffnung für Substrat umfassen. Es kann ein Verschluss für diese Substrateintrittsöffnung vorhanden sein, mit dem die Substrateintrittsöffnung geöffnet und verschlossen werden kann. Ein Zuchtbehälter kann an seiner Unterseite eine Austrittsöffnung für Substrat aufweisen. Es kann ein Verschluss für diese Substrataustrittsöffnung vorhanden sein, mit dem die Substrataustrittsöffnung geöffnet und verschlossen werden kann. Diese Ausgestaltung ermöglicht es, Substrat an der Unterseite zu entnehmen und das entnommene Substrat durch die Substrateintrittsöffnung hindurch wieder hinzuzufügen. Es ist dann auf technisch einfache Weise möglich, das Substrat vollständig durch Schwerkraft an der Unterseite zu entnehmen und anschließend an der Oberseite wieder hinzuzufügen. Es kann so sichergestellt werden, dass für das gesamte Substrat eine übermäßige Verdichtung auf technisch einfache Weise vermieden werden kann. Es kann so erreicht werden, dass das gesamte Substrat während der Anzucht von Insekten wiederholbar befeuchtet werden kann.

Innerhalb eines Zuchtbehälters können ein oder mehrere Luftleitelemente vorhanden sein, mit denen Luft in definierter Weise durch einen Zuchtbehälter hindurchgeleitet werden kann. Der Zuchtbehälter umfasst dann zumindest eine Eintrittsöffnung für Luft, über die Luft in zumindest ein Luftleitelement eingeleitet werden kann. Der Zuchtbehälter umfasst dann zumindest eine Austrittsöffnung für Luft, über die Luft aus zumindest einem Luftleitelement herausgeleitet werden kann. Durch ein Luftleitelement kann das Substrat temperiert werden, um die Anzucht zu optimieren.

Es kann in einer Ausgestaltung der Erfindung die Feuchtigkeit der Luft eingestellt werden. Es kann eine Einrichtung vorhanden sein, mit der die Luftfeuchtigkeit eingestellt werden kann, bevor Luft in den Zuchtbehälter eingeleitet wird. Durch Einstellen der Luftfeuchtigkeit kann der Grad der Verdunstung eingestellt werden und damit der Grad der Temperierung. Außerdem kann bei Bedarf dem Substrat Feuchtigkeit zugeführt werden.

Die Einrichtung, mit der die Luftfeuchtigkeit eingestellt werden kann, kann beispielweise eine Flüssigkeit wie zum Beispiel Wasser vernebeln. Luft kann durch die Einrichtung durch den Nebel hindurchgeleitet und so der Feuchtigkeitsgrad gesteigert werden. Ein Vernebeln kann beispielsweise durch Düsen und/oder durch Ultraschall und/oder durch ein Heizelement erreicht werden. Die Einrichtung kann so eingerichtet sein, dass in der Luft enthaltene Feuchtigkeit kondensieren kann. Es kann so der Feuchtigkeitsgehalt der Luft reduziert werden. Luft kann beispielsweise durch eine gekühlte Lamellenstruktur hindurchgeleitet werden, damit Feuchtigkeit aus der Luft kondensieren kann.

Ein Luftleitelement kann ein Kanal mit einer offenen Seite sein. Die offene Seite des Kanals kann vollständig offen sein. Es ist aber auch möglich, dass die offene Seite des Kanals nur abschnittsweise geöffnet ist. Es ist in jedem Fall zu bevorzugen, dass die Seite überwiegend offen ist. Es können dann Gase, die durch die Anzucht gebildet werden, aus dem Zuchtbehälter abgezogen werden, weil dann Gase zusammen mit Luft die zumindest eine Austrittsöffnung für Luft verlassen können.

Die offene Seite des Kanals kann die Unterseite des Luftleitelements sein. Es kann so vermieden werden, dass das Luftleitelement durch die offene Seite hindurch mit Substratmaterial zugesetzt werden kann.

Es kann auch sein, dass Luft nach einem Eintreten in den Zuchtbehälter durch Substrat hindurchgeleitet wird. Luft kann beispielsweise in einer Ebene durch eine Lufteintrittsöffnung hindurch in den Zuchtbehälter eingeleitet und in Höhe der gleichen Ebene oder einer anderen Ebene durch eine Luftaustrittsöffnung hindurch entnommen werden. Luftleitelemente sind bei dieser Ausgestaltung nicht erforderlich, um zu temperieren und/oder zu entlüften und/oder den Feuchtigkeitsgehalt von Substrat zu regulieren.

Die Oberseite des Luftleitelements ist vorzugsweise vollständig oder zumindest überwiegend geschlossen, um zu vermeiden, dass das Luftleitelement mit Substratmaterial zugesetzt werden kann.

Eine regelmäßige Entnahme von Substrat an der Unterseite eines Zuchtbehälters trägt dazu bei, dass die ein oder mehreren Luftleitelemente nicht durch Öffnungen hindurch zugesetzt werden. Dies gilt insbesondere für den Fall, dass ein Luftleitelement ein Kanal ist, bei dem nur die Unterseite offen ist.

Die Oberseite eines Luftleitelements kann eine Kante sein. Die Kante kann gerundet oder nicht gerundet sein. Es kann so vermieden werden, dass Substrat auf der Oberseite eines Luftleitelements nachteilhaft liegen bleiben kann.

Von der Kante ausgehend kann sich das Luftleitelement nach unten verbreitern. Das Luftleitelement kann beispielsweise im Schnitt wie ein um 180° gedrehtes V sein. Es gibt dann an der Oberseite eine Kante, von der ausgehend sich das Luftleitelement zu beiden Seiten hin kontinuierlich aufweitet. Die beiden Seiten des Kanals werden dann durch Schrägen gebildet. Dies trägt dazu bei, das Substrat schonend und vollständig entnommen werden kann, um so zu einer Optimierung der Anzucht beizutragen. Eine jede Schräge kann mit einer Horizontalen einen Winkel von 35° bis 60° einschließen, um Substrat schonend und vollständig entnehmen zu können.

Es können ein oder mehrere Gebläse für ein Erzeugen einer Luftströmung vorhanden sein, damit Luft durch die ein oder mehreren Luftleitelemente hindurchströmen kann.

Weisen die ein oder mehreren Luftleitelemente ein oder mehrere Öffnungen auf, dann kann im Substrat gespeicherte Flüssigkeit verdunsten und verdunstete Flüssigkeit aus dem Zuchtbehälter heraustransportiert werden. Die Luftleitelemente sind dann Teil der Verdunstungseinrichtung.

Ein Luftleitelement kann von einer Zuchtbehälterwand bis zu einer gegenüberliegenden Zuchtbehälterwand führen. Ein Luftleitelement kann von einer Seitenwand bis zu einer anderen Seitenwand des Zuchtbehälters führen. Ein Luftleitelement kann innerhalb des Zuchtbehälters horizontal verlaufen. Führt ein Luftleitelement von einer Seitenwand zu einer anderen Seitenwand des Zuchtbehälters, so kann das Luftleitelement vermeiden, dass ein übermäßig hoher Druck im Substrat entstehen kann, der den Anzuchterfolg gefährdenden kann. Die ein oder mehreren Luftleitelemente sind dann zugleich Druckschutzelemente. Es können aber auch unabhängig von Luftleitelementen ein oder mehrere Druckschutzelemente vorgesehen sein, was aber den technischen Aufwand erhöht. Verlaufen mehrere Luftleitelemente horizontal innerhalb eines Zuchtbehälters und erstrecken sich Unterseite und Oberseite des Zuchtbehälters ebenfalls horizontal, so kann das Substrat durch eine entsprechende Verteilung der Luftleitelemente besonders gleichmäßig temperiert werden. Gase wie CO₂ können zuverlässig hinreichend vollständig aus dem Zuchtbehälter heraustransportiert werden. Der Zuchtbehälter kann also gleichmäßig entgast werden.

Vorzugsweise sind Luftleitelemente in einer ersten Ebene angeordnet und Luftleitelemente in einer zweiten Ebene und Luftleitelemente in einer dritten Ebene usw. Die Ebenen können parallel zu einander angeordnet sein. Die Abstände zwischen den Ebenen können gleich sein. Die Abstände zwischen Luftleitelementen einer Ebene können gleich sein. Es ist so möglich, einen Zuchtbehälter gleichmäßig zu belüften und damit gleichmäßig zu temperieren und gleichmäßig zu entgasen.

Vorzugsweise sind die Luftleitelemente einer Ebene relativ zu Luftleitelementen einer darunter und/oder darüber liegenden Ebene versetzt zueinander angeordnet. Diese Anordnung hat sich als günstig herausgestellt, um übermäßig großen Verdichtungen entgegenzuwirken und zwar vor allem auch in Kombination mit einer Entnahme von Substrat an der Zuchtbehälterunterseite und Rückführung des entnommenen Substrats über die Oberseite des Zuchtbehälters. Luftleitelemente von zwei Ebenen, zwischen denen sich eine Ebene mit Luftleitelementen befindet, können zueinander nicht versetzt angeordnet sein, um den Zuchtbehälter beispielsweise besonders gleichmäßig temperieren und/oder entgasen zu können.

Ein Luftleitelement kann maximal 4 m oder maximal 3 m lang sein. Die maximale Breite eines Luftleitelements kann 1000 mm sein. Die minimale Breite eines Luftleitelements kann 10 mm sein. Die maximale Höhe eines Luftleitelements kann 1000 mm sein. Die minimale Höhe eines Luftleitelements kann 10 mm sein. Die maximale Stärke einer jeden Wand des Luftleitelements kann wenigstens 20 mm betragen. Die minimale Stärke einer jeden Wand des Luftleitelements kann wenigstens 0.5 mm betragen. Ein so dimensioniertes, an einer Seite offenes Luftleitelement macht es möglich, dass Luft durch das Luftleitelement für ein geeignetes Temperieren des Substrats hindurchströmen kann, ohne dass das Substrat nachteilhaft aufgewirbelt werden kann. Ein Aufwirbeln des Substrats beeinflusst nachteilhaft den Anzuchterfolg, wie Versuche gezeigt haben. Luft sollte daher nicht zu schnell durch ein Luftleitelement. Die Luftmenge, die hindurchströmt, sollte auf der anderen Seite groß genug sein, um Flüssigkeit für ein Kühlen hinreichend verdunsten zu können und um durch die Anzucht gebildete Gase wie CO₂ hinreichend vollständig aus dem Zuchtbehälter heraustransportieren zu können.

Ein Zuchtbehälter kann zwei Eintrittsöffnungen für Luft aufweisen. Die eine Eintrittsöffnung kann an einer Seitenwand des Zuchtbehälters vorhanden sein und die andere Seite an einer anderen Seitenwand, so zum Beispiel an einer gegenüberliegenden Seitenwand des Zuchtbehälters. Es kann dann Luft vorteilhaft von zwei verschiedenen Seiten aus in den Zuchtbehälter eingeleitet werden.

Ein Zuchtbehälter kann an zwei gegenüberliegenden Seitenwänden jeweils wenigstens eine Eintrittsöffnung und jeweils wenigstens eine Austrittsöffnung für Luft aufweisen. Dies trägt dazu bei, auf technisch einfache Weise den Zuchtbehälter geeignet belüften zu können.

Aus konstruktiven Gründen ist zu bevorzugen, dass eine solche Seitenwand genau eine Eintrittsöffnung für Luft aufweist und zwei Austrittsöffnungen für Luft. Die Eintrittsöffnung für Luft kann sich dann zwischen den beiden Austrittsöffnungen für Luft befinden. Luft kann beispielsweise von einer Eintrittsöffnung über Luftleitelemente zu den beiden gegenüberliegenden Austrittsöffnungen strömen und über die beiden gegenüberliegenden Austrittsöffnungen aus dem Zuchtbehälter herausgeleitet werden.

Eine Eintrittsöffnung für Luft kann kreisrund oder eckig sein. Eine Austrittsöffnung für Luft kann rechteckförmig sein. Die Luftaustrittsöffnung kann kreisrund sein. Eine Austrittsöffnung für Luft kann vorteilhaft schlitzförmig oder anders langgestreckt sein. Es ist dann vorteilhaft möglich, Luft von Luftleitelementen zu sammeln und in gesammelter Form einer Austrittsöffnung zuzuleiten, um die Luft dann auf technisch einfache Weise mit geringen Druckverlusten beispielsweise einer Wiederaufbereitung zuführen zu können. Die Zuchtvorrichtung kann also auch eine Luftaufbereitungsvorrichtung umfassen. Die Luftaufbereitungsvorrichtung kann ein oder mehrere Filter und/oder eine Zentrifuge umfassen, um Partikel und/oder Gerüche aus der Luft herauszufiltern bzw. zu entfernen.

Hinter einer Eintrittsöffnung für Luft kann sich eine Behälterzwischenwand befinden. Die Behälterzwischenwand kann eine Vielzahl von Löchern aufweisen. Ein jedes Loch kann in ein Luftleitelement einmünden. Strömt Luft über die Eintrittsöffnung in den Zuchtbehälter hinein, so befindet sich die Luft in einem Zwischenraum, der durch die Wand mit der Eintrittsöffnung und die Zwischenwand mit den Löchern begrenzt wird. Luft strömt dann weiter durch die Löcher hindurch. Im Anschluss daran wird die strömende Luft durch die Luftleitelemente zur Austrittsseite für die Luft gelenkt.

Um Luft in den Zuchtbehälter einzuleiten, kann ein jedes Luftleitelement unmittelbar mit einem Schlauch oder Rohr verbunden sein. Über das Rohr bzw. über den Schlauch kann Luft zugeführt werden. Dadurch wird der Druckverlust geringgehalten. In gleicher Weise kann Luft aus dem Zuchtbehälter mit geringem Druckverlust herausgeleitet werden. Es sind dann also an der Luftaustrittsseite eines Luftleitelements Schläuche oder Rohre angeschlossen.

Die Seitenwand des Zuchtbehälters kann zumindest überwiegend gewölbt oder auf andere Weise zumindest überwiegend beispielsweise wie ein Trichter geformt sein. Die Seitenwand des Zuchtbehälters, die eine Eintrittsöffnung für Luft umfasst, kann dann also nach außen vorstehen. Die Zwischenwand kann eben sein, damit die nach außen vorstehende Seitenwand und die Zwischenwand einen Raum für Luft einschließen können. Umgekehrt kann die Seitenwand des Zuchtbehälters eben sein und die Zwischenwand kann nach innen vorstehen, damit Seitenwand und Zwischenwand einen Raum für Luft einschließen können. Der Fall mit der ebenen Zwischenwand ist aber aus Fertigungsgründen zu bevorzugen. Es kommt hinzu, dass eine nach innen vorstehende Zwischenwand bewirken kann, dass Substrat nachteilhaft verdichtet wird. Es kann auch sein, dass Zwischenwand und Seitenwand nicht eben sind, um einen Raum einzuschließen, durch den Luft auf Luftleitelemente verteilt werden kann.

Die Luftleitelemente können in einen Abluftkanal einmünden. Der Abluftkanal kann Löcher aufweisen, durch die hindurch Luft aus Luftleitelementen in den Abluftkanal hineinströmen kann. Der Abluftkanal kann die Luft aus den Luftleitelementen sammeln und weiter zu ein oder mehreren Luftaustrittsöffnungen leiten, über die Luft aus dem Zuchtbehälter herausgeleitet wird. Beispielsweise ein oder zwei Enden des Abluftkanals können geöffnet sein, damit Luft über ein oder zwei Enden zu ein oder zwei Luftaustrittsöffnungen strömen kann. Der Abluftkanal kann darüber hinaus vollständig geschlossen sein. Der Abluftkanal kann an einer Seitenwand oder einer Zwischenwand des Zuchtbehälters befestigt sein.

Eine oberste, erste Reihe der Löcher kann in Luftleitelemente einer ersten Ebene einmünden. Eine unmittelbar darunter befindliche, zweite Reihe der Löcher kann in Luftleitelemente einer dritten Ebene einmünden. Zwischen der ersten Ebene und der dritten Ebene befindet sich dann noch eine zweite Ebene mit Luftleitelementen. Eine unmittelbar unter der zweiten Reihe befindliche, dritte Reihe der Löcher kann in Luftleitelemente einer fünften Ebene einmünden. Diese Ausgestaltung ist insbesondere dann von Vorteil, wenn es noch eine zweite Eintrittsöffnung für Luft gibt. Die gegenläufigen Luftströme, die benachbart sind, können besonders homogen dem Substrat Feuchtigkeit für ein Kühlen entziehen. Es kann damit besonders gelichmäßig gekühlt werden.

Jeweils ein Abluftkanal kann auf Höhe der ersten, dritten und fünften Ebene angeordnet sein, in die die Luftleitelemente der ersten, dritten und fünften Ebene einmünden. Ein jeder Abluftkanal kann zu einer gemeinsam genutzten Luftaustrittsöffnung führen. Eine langgestreckte Luftaustrittsöffnung ist dann aus Gründen einer einfachen Fertigung mit geringem Bauraum zu bevorzugen. Eine langgestreckte Austrittsöffnung kann wie ein Schlitz geformt sein. Die langgestreckte Austrittsöffnung kann wie ein Rechteckeck geformt sein. Eine langgestreckte Austrittsöffnung würde sich dann beispielsweise von der obersten (ersten) Ebene bis zur untersten (fünften) Ebene erstrecken. Um den Strömungswiderstand für Luft gering zu halten, können beide Enden eines jeden Abluftkanals in eine Austrittsöffnung einmünden. Beide Austrittsöffnungen sind dann vorzugsweise langgestreckt, damit nur jeweils eine Austrittsöffnung bei den Enden der Luftsammelkanäle vorhanden sein und gefertigt werden muss.

Der Seitenwand mit der Lufteintrittsöffnung kann eine Seitenwand mit einer zweiten Eintrittsöffnung für Luft gegenüberliegen. Die gegenüberliegende Seitenwand kann mit einer weiteren Zwischenwand einen Raum einschließen. Die Behälterzwischenwand kann eine Vielzahl von Löchern aufweisen. Ein jedes Loch kann in ein Luftleitelement einmünden. Strömt Luft über die zweite Eintrittsöffnung in den Zuchtbehälter hinein, so befindet sich die Luft in einem Zwischenraum, der durch die Wand mit der zweiten Eintrittsöffnung und die weitere Zwischenwand mit den Löchern begrenzt wird. Luft strömt dann weiter durch die Löcher hindurch. Im Anschluss daran wird die strömende Luft durch die Luftleitelemente zur Austrittsseite für die Luft gelenkt.

Eine oberste, erste Reihe der Löcher kann in Luftleitelemente einer zweiten Ebene einmünden. Eine unmittelbar darunter befindliche, zweite Reihe der Löcher kann in Luftleitelemente einer vierten Ebene einmünden.

Jeweils ein Abluftkanal kann auf Höhe der zweiten und vierten Ebene angeordnet sein, in die die Luftleitelemente der zweiten, vierten und sechsten Ebene einmünden. Ein jeder Abluftkanal kann zu einer gemeinsam genutzten, langgestreckten Austrittsöffnung oder zu zwei langgestreckten Austrittsöffnungen führen.

Es können auch mehr oder weniger als fünf oder sechs Ebenen vorhanden sein. Ein jeder Abluftkanal kann eine eigene Luftaustrittsöffnung oder zwei eigene Luftaustrittsöffnungen haben.

Die Befeuchtungseinrichtung kann so angeordnet sein, dass das entnommene Substrat unmittelbar nach dem Befeuchten zurück in einen gewünschten Zuchtbehälter gebracht wird. Die Befeuchtungseinrichtung kann daher zweckmäßig oberhalb von ein oder mehreren Zuchtbehältern der Zuchtvorrichtung angeordnet sein. Die Befeuchtungseinrichtung kann ein oder mehrere Düsen oder andere Austrittsöffnungen für Flüssigkeit umfassen, aus denen während des Betriebs Flüssigkeit austritt, um Substrat zu befeuchten. Die ein oder mehreren Düsen oder anderen Flüssigkeits-Austrittsöffnungen können so ausgewählt sein, dass Flüssigkeit zerstäubt wird, damit entnommenes Substrat zunächst nur oberflächlich befeuchtet wird. Zunächst nur eine oberste Schicht von entnommenem Substrat anzufeuchten, reduziert den Stress für die Insektenlarven und verbessert daher den Anzuchterfolg.

Düsen oder andere Austrittsöffnungen für Flüssigkeit können entlang einer geraden Linie angeordnet sein, die quer zur Transportrichtung des entnommenen Substrats verläuft. Alternativ kann eine schlitzförmige Austrittsöffnung für Flüssigkeit vorgesehen sein, die quer zur Transportrichtung des entnommenen Substrats verläuft. Weiter verbessert wird das Substrat besonders schonend befeuchtet.

Die Befeuchtungseinrichtung kann so eingerichtet sein, dass schrittweise mit zeitlichem Abstand zwischen den Schritten befeuchtet wird. Substrat wird dann in einem ersten Schritt befeuchtet. Es vergeht dann eine gewisse Zeit, ehe das entnommene und bereits ein erstes Mal befeuchtete Substrat ein zweites Mal befeuchtet wird. Es kann wieder Zeit vergehen, ehe ein drittes Mal befeuchtet wird. Auch dies trägt dazu bei, besonders schonend zu befeuchten und damit den Zuchterfolg zu verbessern. Die gewisse Zeit zwischen zwei Schritten kann wenigstens 3 Sekunden oder wenigstens 5 Sekunden oder wenigstens 10 Sekunden lang sein.

Um schrittweise besonders schonend zu befeuchten, können Düsen oder andere Flüssigkeits-Austrittsöffnungen in Transportrichtung des entnommenen Substrats gesehen räumlich hintereinander angeordnet sein. Wird entnommenes Substrat transportiert, so wird in zeitlichen Abständen befeuchtet und damit besonders schonend.

Die Rücktransporteinrichtung kann so eingerichtet sein, dass diese entnommenes Substrat zwischen zwei Befeuchtungsschritten mischt. Es wird dann also entnommenes Substrat befeuchtet, danach gemischt und danach wieder befeuchtet. Es wird so verbessert erreicht, dass entnommenes Substrat schonend und homogen befeuchtet wird. Die Rücktransporteinrichtung kann so eingerichtet sein, dass entnommenes Substrat zwischen zwei Befeuchtungsschritten von einer Transportebene auf eine andere, darunter liegende Transportebene fällt. Durch das Fallen gelingt das Mischen. Ein Mischen kann aber auch anders erfolgen, so zum Beispiel mit einem langsam drehenden Rührwerkzeug oder einer Rutsche mit geeignet unebener Oberfläche.

Die Rücktransporteinrichtung und die Befeuchtungseinrichtung können so eingerichtet sein, dass nach einem Mischen Zeit vergeht, ehe entnommenes Substrat befeuchtet wird. Dies kann erreicht werden, indem entnommenes Substrat zunächst entlang einer horizontalen Ebene hinreichend langsam zu wenigstens einer Düse oder wenigstens einer anderen Austrittsöffnungen für Flüssigkeit transportiert wird. Es wird so ausgenutzt, dass Insektenlarven in Substrat hineinkriechen und Kot aufgrund seiner großen Dichte nach unten sinkt. Es befinden sich dann im Idealfall nur noch Futterstoffe auf der Oberseite des entnommenen Substrats, so dass nur noch Futterstoffe unmittelbar befeuchtet werden. Auch dadurch kann der Anzuchterfolg verbessert werden.

Die Rücktransporteinrichtung kann so eingerichtet sein, dass Substrat entlang einer horizontalen Ebene durch eine Platte transportiert wird, die sich zunächst langsam in Transportrichtung bewegt und dann schnell zurück. Es wird so das Trägheitsverhalten von Masse ausgenutzt, um Substrat besonders schonend in eine gewünschte Richtung zu transportieren. Es sind dann keine seitlichen Begrenzungen des Transportwegs erforderlich, durch die entnommenes Substrat nachteilhaft verdichtet werden könnte. Die Platte kann beispielsweise aus Metall oder Kunststoff bestehen.

Die Rücktransporteinrichtung kann so eingerichtet sein, dass entnommenes Substrat von einer für den horizontalen Transport vorgesehenen Platte auf eine tieferliegende Platte fällt, wenn entnommenes Substrat über die erstgenannte Platte hinaus transportiert worden ist. Besonders schonend kann so entnommenes Substrat gemischt und/oder umgewälzt werden. Es sind beispielsweise keine Abstreifer erforderlich, die Insektenlarven beschädigen könnten.

In Transportrichtung gesehen kann beim Ende von Platten zumindest eine Düse oder zumindest eine andere Austrittsöffnung für Flüssigkeit vorgesehen sein, um entnommenes Substrat zu befeuchten.

Die Zuchtvorrichtung kann eine Steuereinrichtung umfassen, mit der die Flüssigkeitszufuhr zu entnommenem Substrat und damit die Kühlung gesteuert wird. Die Flüssigkeitszufuhr und damit die Kühlung können in Abhängigkeit von einer gemessenen Temperatur gesteuert werden. Es kann sich um eine Temperatur handeln, die durch einen im Zuchtbehälter angeordneten Temperatursensor gemessen wird. Es kann sich um eine Temperatur handeln, die durch einen an einer Luftaustrittsöffnung eines Zuchtbehälters angeordneten Temperatursensor gemessen wird. Es kann so in Abhängigkeit vom Kühlungsbedarf für einen Zuchtbehälter die Flüssigkeitszufuhr zum entnommenen Substrat gesteuert werden. Es kann sich um eine Temperatur handeln, die durch einen an einer Lufteintrittsöffnung eines Zuchtbehälters angeordneten Temperatursensor gemessen wird. Es kann dadurch berücksichtigt werden, mit welcher Temperatur Substrat im Zuchtbehälter aufgrund von Lufttemperatur gekühlt oder erwärmt wird, umso genauer den Kühlbedarf durch Verdunstungskälte und damit den Flüssigkeitsbedarf ermitteln zu können.

Durch die Steuereinrichtung kann beispielsweise das Luftvolumen, die Lufttemperatur und/oder die Feuchtigkeit gesteuert werden. Durch die Steuereinrichtung kann beispielsweise das Volumen an entnommenem Substrat, und/oder die Geschwindigkeit des daraus resultierenden Substratflusses und/oder die Frequenz der Entnahme von Substrat gesteuert werden. Es können Parameter wie Flüssigkeitszufuhr oder Luftvolumen gleichzeitig gesteuert werden oder nacheinander.

Die Flüssigkeitszufuhr und damit die Kühlung kann in Abhängigkeit von einer gemessenen Feuchtigkeit gesteuert werden. Es kann sich um eine Feuchtigkeit handeln, die durch einen im Zuchtbehälter angeordneten Feuchtigkeitssensor gemessen wird. Es kann sich um eine Feuchtigkeit handeln, die durch einen an einer Luftaustrittsöffnung eines Zuchtbehälters angeordneten Feuchtigkeitssensor gemessen wird. Es kann so unter Berücksichtigung des Feuchtegehalts des Substrats im Zuchtbehälter die Flüssigkeitszufuhr zum entnommenen Substrat und damit die Kühlung verbessert gesteuert werden. Es kann sich um eine Feuchtigkeit handeln, die durch einen an einer Lufteintrittsöffnung eines Zuchtbehälters angeordneten Feuchtigkeitssensor gemessen wird. Es kann dadurch der Feuchtegehalt von eintretender Luft berücksichtigt werden, die den Grad der Verdunstung im Zuchtbehälter und damit das Kühlvermögen durch Verdunstungskälte beeinflusst. Es kann auch dadurch verbessert der Flüssigkeitsbedarf für entnommenes Substrat bestimmt werden.

Entsprechendes gilt auch für den Fall, dass auf andere Weise gekühlt wird, so zum Beispiel durch einen im Zuchtbehälter angeordneten Wärmetauscher. Auch dann kann die Kühlung in Abhängigkeit von ein oder mehreren der vorgenannten Messwerte gesteuert werden, um die Anzucht zu optimieren.

Die Flüssigkeitszufuhr und damit die Kühlung können in Abhängigkeit von der Größe und dem Alter von Insektenlarven gesteuert werden. Sind die Insektenlarven klein, so benötigen diese mehr Wärme. Entsprechend gering ist der Bedarf für eine Kühlung. Eine Kühlung durch Verdunstungskälte und/oder eine Kühlung auf andere Weise sind entsprechend gering zu halten. Je größer die Insektenlarven sind, um so größer ist der Bedarf für Kühlung in einem Zuchtbehälter. Die Flüssigkeitszufuhr und damit die Kühlung durch Verdunstungskälte sind entsprechend zu steigern. Dies gilt entsprechend, wenn auf andere Weise gekühlt wird.

Es kann bei kleinen Insektenlarven sogar erforderlich sein, Wärme zuzuführen, so zum Beispiel durch Zufuhr von erwärmter Luft oder durch Wärmezufuhr über einen im Zuchtbehälter angeordneten Wärmetauscher. Die Steuereinrichtung kann so eingerichtet sein, dass diese eine Wärmezufuhr in Abhängigkeit von der Größe von Insektenlarven steuert.

Da die Wachstumsrate von Insektenlarven vorhersagbar ist, kann es für eine Steuerung in Abhängigkeit von der Größe von Insektenlarven genügen, dass der Steuereinrichtung nur anfänglich mitgeteilt wird, wenn eine Anzucht begonnen wird. Alternativ kann dies durch eine Steuereinrichtung auch automatisiert ermittelt werden, so zum Beispiel durch automatisiertes Bestimmen des spezifischen Gewichts von Substrat mit Hilfe einer dafür geeigneten Messeinrichtung oder mithilfe einer Kamera und eine Bildauswertungssoftware.

Alternativ oder ergänzend kann die Steuereinrichtung die Luftzufuhr und damit die Kühlung durch Verdunstungskälte zu einem Zuchtbehälter der Zuchtvorrichtung steuern. Dies kann in Abhängigkeit von einem ermittelten Kühlbedarf und/oder in Abhängigkeit von zuvor beschriebenen ein oder mehreren Feuchtemessungen und/oder in Abhängigkeit von der Größe von Insektenlarven wie zuvor beschrieben geschehen.

Die Luftzufuhr zu einem Zuchtbehälter kann in Abhängigkeit von der Höhe der Luftzufuhr erfolgen. Wird Substrat an der Unterseite eines Zuchtbehälters entnommen, befeuchtet und dann über die Zuchtbehälteroberseite zurück in den Zuchtbehälter gebracht, so hat dies zur Folge, dass der Feuchtigkeitsgehalt des Substrats von seiner Höhe im Zuchtbehälter abhängt. Je weiter unten das Substrat in einem Zuchtbehälter ist, umso geringer ist sein Feuchtigkeitsgehalt aufgrund der zuvor angewendeten Kühlung durch Verdunstungskälte. Es kann daher erforderlich sein, dass die Luftzufuhr in einem unteren Bereich eines Zuchtbehälters größer sein muss als in einem oberen Bereich des Zuchtbehälters, wenn gleichmäßig gekühlt werden soll, um so die Anzucht zu optimieren. Es ist daher von Vorteil, wenn die Luftzufuhr zu einem Zuchtbehälter in Abhängigkeit von der Höhe der Luftzufuhr erfolgt. Der Zuchtbehälter kann dann mehrere Lufteintrittsöffnungen aufweisen, die in unterschiedlichen Höhen angeordnet sind. Zwei oder mehr Lufteintrittsöffnungen können übereinander entlang einer Senkrechten angeordnet sein. Es kann dann sein, dass grundsätzlich die Luftzufuhr in eine obenliegende Eintrittsöffnung für Luft geringer ist als in eine untenliegende Eintrittsöffnung für Luft. Die Zuchtvorrichtung kann so eingerichtet sein, dass die Luftzufuhr in fest vorgegebener Weise von der Höhe abhängt. Die Zuchtvorrichtung kann so eingerichtet sein, dass die Luftzufuhr in Abhängigkeit von der Höhe durch die Steuereinrichtung gesteuert wird.

Die Zuchtvorrichtung kann eine Mehrzahl von Zuchtbehältern umfassen. Die Zuchtvorrichtung kann eine Wärmetauschereinrichtung umfassen, mit der Wärme zwischen Zuchtbehältern ausgetauscht werden kann. Gibt es eine Mehrzahl von Zuchtbehältern, so können sich in einem ersten Zuchtbehälter kleine Insektenlarven befinden und in einem zweiten Zuchtbehälter große Insektenlarven. Es kann sein, dass der erste Zuchtbehälter mit den kleinen Insektenlarven erwärmt werden muss und der zweite Zuchtbehälter mit den großen Insektenlarven gekühlt werden muss. Durch den Wärmetauscher kann dann Wärme zwischen dem ersten und dem zweiten Zuchtbehälter so ausgetauscht werden, dass der erste Zuchtbehälter dadurch erwärmt und der zweite Zuchtbehälter dadurch gekühlt wird. Es kann so der für die Aufzucht benötigte Energiebedarf geringgehalten werden.

Die Rücktransporteinrichtung kann ein Becherwerk, insbesondere ein Pendelbecherwerk, umfassen, um entnommenes Substrat von unten nach oben transportieren zu können. Entnommenes Substrat wird über eine Vielzahl von Bechern transportiert. So wird vermieden, das Substrat während des Transports nachteilhaft einem übermäßig großen Druck ausgesetzt wird.

Ein Becherwerk kann eine doppelte oder zentrale, umlaufende Kette oder ein oder zwei Gurte umfassen. An den ein oder zwei Ketten bzw. Gurten sind Zuchtbehälter hintereinander befestigt, die Becher genannt werden. Die Becher können im Schnitt beispielsweise V-förmig oder U-förmig sein. Die Becher können aus Metall wie zum Beispiel Stahl oder Kunststoff bestehen. Eine Kette kann beispielsweise aus Metall bestehen. Auch ein Gurt kann aus Metall gebildet sein. Beispielsweise über eine Rutsche kann entnommenes Substrat in die Becher gefüllt werden. Werden die Kette oder Ketten bzw. Gurt oder Gurte durch einen Motor angetrieben, so werden die Becher dadurch transportiert. Am Ziel angekommen, können Becher gewendet und so entladen werden. Substrat kann so auf einen anderen Teil der Rücktransporteinrichtung abgeladen werden, so zum Beispiel auf eine Platte oder ein Förderband.

Im Fall eines Pendelbecherwerks sind die Becher hintereinander verschwenkbar an umlaufenden ein oder zwei Ketten oder ein oder zwei Gurten angebracht.

Das Becherwerk bzw. Pendelbecherwerk kann für den Rücktransport von entnommen Substrat für mehrere Zuchtbehälter genutzt werden.

Die Rücktransporteinrichtung kann oberhalb von ein oder mehreren Zuchtbehältern ein oder mehrere weitere Fördereinrichtungen umfassen, mit denen entnommenes Substrat zu Substrateintrittsöffnungen von gewünschten Zuchtbehältern transportiert werden kann. Eine weitere Fördereinrichtung kann oberhalb einer Substrateintrittsöffnung eine Verteilereinrichtung umfassen, mit der entnommenes Substrat in einen gewünschten Zuchtbehälter gebracht werden kann und dabei räumlich verteilt werden kann. Das Substrat wird dann nicht immer an die gleiche Stelle in den Zuchtbehälter von oben eingefüllt, sondern immer wieder an eine andere Stelle, um Insektenlarven nicht durch einen zu großen Druck zu beschädigen. Zudem dient es dazu, das Substrat flächendeckend gleichmäßig zu verteilen. Die Verteilereinrichtung kann ein drehbares Rohr umfassen, das beispielsweise durch einen Motor gedreht werden kann. Das Rohr verläuft bogenförmig derart, dass durch das Drehen entnommenes Substrat räumlich verteilt werden kann, wenn es aus dem bogenförmigen Rohr austritt.

Die Entnahmeeinrichtung kann eine Mehrzahl von Rinnen oder Stutzen aufweisen, über die Substrat aus dem Zuchtbehälter herausgeleitet werden kann. In Aufsicht gesehen kann eine jede Rinne bzw. ein jeder Stutzen zwischen Luftleitelementen angeordnet sein. Eine Öffnung, über die Substrat die Entnahmeeinrichtung verlassen kann, kann mit einer Abdeckung versehen sein. Die Abdeckung kann für ein Schonen des Substrats einen Abstand zur Öffnung aufweisen. Der Abstand kann nicht mehr als 5 cm oder nicht mehr als 3 cm sein. Der Abstand kann wenigstens 0.2 cm oder wenigstens 2 cm betragen. Die Abdeckung kann rotierbar gelagert sein, um die Öffnung durch ein Rotieren das Substrat schonend öffnen und verschließen zu können.

Ein Zuchtbehälter besteht vorzugsweise aus einer Mehrzahl von Behältermodulen. Ein jedes Behältermodul kann beispielsweise 2 m x 2 m x 1,5 m sein oder 3 m x 4 m x 2 m dimensioniert sein (B x L x H). Ein Behältermodul kann auf ein zweites Behältermodul gesetzt werden, um einen Zuchtbehälter zu bilden. Die Höhe eines Zuchtbehälters hängt damit von der Zahl seiner Behältermodule ab. Es können so vorteilhaft Zuchtbehälter mit unterschiedlichen Höhen sehr flexibel und einfach bereitgestellt werden. Außerdem ist der Fertigungsaufwand gering.

Ein Zuchtbehälter kann eine Grundfläche von wenigstens 1 m² oder von wenigstens 4 m² oder von wenigstens 8 m² aufweisen. Ein Zuchtbehälter kann eine Grundfläche von nicht mehr als 25 m² oder nicht mehr als 20 m² oder nicht mehr als 15 m² aufweisen. Ein Zuchtbehälter kann eine maximale Tiefe von 4 m oder von 3 m aufweisen, um geeignet Temperieren zu können. Ein Zuchtbehälter kann eine minimale Tiefe von 1 m oder von 2 m aufweisen. Ein Zuchtbehälter kann wenigstens 1 m oder wenigstens 5 m oder wenigstens 10 m hoch sein. Ein Zuchtbehälter kann nicht mehr als 40 m oder nicht mehr als 35 m oder nicht mehr als 30 m hoch sein.

Ein Zuchtbehälter kann ganz oder teilweise aus Metall bestehen, so zum Beispiel aus einem korrosionsbeständigen Stahl. Ein Zuchtbehälter kann ganz oder teilweise aus Kunststoff bestehen. Luftleitelemente im Zuchtbehälter können vollständig oder teilweise aus Metall oder Kunststoff bestehen.

Die Zuchtvorrichtung kann eine Trenneinrichtung für ein Ernten von Insektenlarven umfassen. Die Trenneinrichtung kann so eingerichtet sein, dass Insektenlarven von dem übrigen Teil des Substrats getrennt werden können. Die Trenneinrichtung kann so eingerichtet sein, dass Kot von dem übrigen Teil des Substrats getrennt werden kann. Die Trenneinrichtung kann so eingerichtet sein, dass Futter von dem übrigen Teil des Substrats getrennt werden kann. Die Trenneinrichtung kann so eingerichtet sein, dass in einem ersten Schritt Insektenlarven von dem übrigen Teil des Substrats getrennt werden. In einem zweiten Schritt wird Futter von dem verbliebenen Teil des Substrats getrennt. Das herausgetrennte Futter kann für das Füttern von anderen Insektenlarven verwendet werden, was vorzugsweise automatisiert geschehen kann. Die Trenneinrichtung kann ein Sieb umfassen, durch das Larven aus dem übrigen Teil des Substrats herausgesiebt werden können. Die Trenneinrichtung kann ein Sieb umfassen, durch das Futter aus dem verbliebenen übrigen Teil des Substrats herausgesiebt werden kann.

Einem Zuchtbehälter kann zu Beginn einer Anzucht zunächst Wärme zugeführt werden, solange Insektenlarven noch klein sind. Dies kann bis zu 8 oder 9 Tage dauern. Danach ist es in der Regel erforderlich, einen Zuchtbehälter zu kühlen.

Um einen Zuchtbehälter effizient erwärmen zu können, kann Wärme von einem Zuchtbehälter mit großen Insektenlarven zu einem Zuchtbehälter mit kleinen Insektenlarven transportiert werden. Es kann daher ein Wärmetauscher vorgesehen sein, über den Wärme zwischen Zuchtbehältern ausgetauscht werden kann. Ein Wärmetausch kann auch stattfinden, indem Luft, die einen Zuchtbehälter mit großen Insektenlarven durchströmt hat, in einen Zuchtbehälter mit kleinen Insektenlarven eingeleitet wird.

Ein Zuchtbehälter kann ein oder mehrere Temperatursensoren umfassen. Ein Temperatursensor kann bei der Substrateintrittsöffnung vorhanden sein. Ein Temperatursensor kann bei der Substrataustrittsöffnung vorhanden sein. Die ein oder mehreren Temperatursensoren können zur Steuerung der Temperierung des Zuchtbehälters genutzt werden.

Ein Zuchtbehälter kann ein oder mehrere Feuchtigkeitssensoren umfassen. Ein Feuchtigkeitssensor kann bei der Substrateintrittsöffnung vorhanden sein. Ein Feuchtigkeitssensor kann bei der Substrataustrittsöffnung vorhanden sein. Aus der Differenz der beiden Feuchtigkeitsmessungen kann dann auf die Menge an Flüssigkeit geschlossen werden, die verdunstet worden ist. Die ein oder mehreren Feuchtigkeitssensoren können zur Steuerung der Temperierung des Zuchtbehälters und/ oder zur Steuerung der Befeuchtung von entnommenen Substrat genutzt werden.

Die Erfindung betrifft außerdem ein System umfassend eine Mühle zur Verarbeitung von Getreide wie zum Beispiel Weizen und eine erfindungsgemäße Zuchtvorrichtung. Die Dimension der Zuchtvorrichtung ist so an die Dimension der Mühle angepasst, dass die Mühlennachprodukte der Mühle insbesondere praktisch vollständig oder wenigsten zu 50% oder wenigsten zu 70% oder wenigsten zu 90% als Futter für die Anzucht von Insektenlarven genutzt werden können.

Die Erfindung betrifft auch ein Verfahren für ein Anzüchten von Insektenlarven mit einer zuvor beschriebenen Zuchtvorrichtung.

Substrat umfassend Insektenlarven und Futter für die Insektenlarven wird in einen Zuchtbehälter der Zuchtvorrichtung gegeben.

Innerhalb von fünf Tagen oder innerhalb von zwei Tagen oder innerhalb von 12 Stunden wird das Substrat von der Unterseite des Zuchtbehälters vollständig oder zu wenigstens 80% oder zu wenigstens 90% entnommen und über die Oberseite des Zuchtbehälters wieder in den Zuchtbehälter gebracht. Innerhalb von fünf Tagen oder innerhalb von zwei Tagen oder innerhalb von 12 Stunden wird das Substrat vorzugsweise kein zweites Mal von der Unterseite des Zuchtbehälters vollständig oder zu wenigstens 80% oder zu wenigstens 90% entnommen und über die Oberseite des Zuchtbehälters wieder in den Zuchtbehälter gebracht.

Das Substrat kann aufgrund von Schwerkraft den Zuchtbehälter von Substrateintrittsöffnung zur Substrataustrittsöffnung passieren.

Nach Ablauf der 12 Stunden bis fünf Tagen kann die Entnahme wiederholt werden. Eine Entnahme von Substrat aus dem Zuchtbehälter kann schrittweise erfolgen. Es kann also erst eine erste Charge an Substrat entnommen werden. Es kann dann eine zeitliche Pause eingelegt werden, ehe eine nächste Charge entnommen wird. Nach beispielsweise 12 Stunden, drei oder fünf Tagen kann dann Substrat vollständig oder zu wenigstens 80% oder zu wenigstens 90% entnommen und dem Zuchtbehälter wieder zugeführt worden sein. Eine schrittweise Entnahme, die gleichmäßig über einen gewünschten Zeitraum verteilt wird, ist zu bevorzugen, um unerwünscht hohen Verdichtungen besonders zuverlässig entgegenzuwirken. Es kann alternativ aus gleichem Grund kontinuierlich oder quasikontinuierlich entnommen werden und dann so, dass sich die Entnahme über den gewünschten Zeitraum von beispielsweise ein bis fünf Tagen verteilt. Wird schrittweise entnommen, so können zwischen zwei Schritten eine Pause von wenigstens einer Stunde oder wenigstens zwei Stunden liegen. Die Pause kann nicht mehr als 48 Stunden oder nicht mehr als 12 Stunden oder nicht mehr als 4 Stunden lang sein.

Bei einer schrittweisen Entnahme ist es vorteilhaft möglich, erst einem ersten Zuchtbehälter eine Charge an Substrat an der Unterseite zu entnehmen und diese Charge wieder in den ersten Zuchtbehälter auf der Oberseite zurückzubringen. Es wird dann einem zweiten Zuchtbehälter eine Charge an Substrat an der Unterseite entnommen und diese Charge wieder in den zweiten Zuchtbehälter über die Oberseite zugeführt. Erst danach wird eine zweite Charge dem ersten Zuchtbehälter wieder an der Unterseite entnommen und wieder über die Oberseite zurückgebracht. Es wird also Substrat schrittweise dem einen Zuchtbehälter entnommen und wieder zurückgeführt und zwischen zwei Schritten einem anderen Zuchtbehälter Substrat entnommen und wieder zurückgeführt. Es ist so möglich, eine Vielzahl von Zuchtbehältern zur Anzucht parallel zu verwenden und das mit geringem technischem Aufwand, weil die Zahl an benötigen Fördereinrichtungen geringgehalten werden kann.

Das Substrat kann ein Mühlennachprodukt umfassen. Das Substrat kann Kleie und/oder Mehl umfassen. Das Substrat kann Mineralien und/oder Aminosäuren umfassen. Substrat kann Mühlenebenprodukte umfassen. Insektenlarven im Substrat produzieren während der Anzucht Kot, der dann ebenfalls im Substrat enthalten sein kann. Das Substrat kann ein Schüttgut sein.

Entnommenes Substrat kann nach der Entnahme befeuchtet werden. Durch das Befeuchten kann die Feuchtigkeit von entnommenem Substrat beispielsweise um 5% bis 30% gesteigert werden, so zum Beispiel von 20 bis 30 % auf 40 bis 50%. 1 bis 5 Liter Wasser pro m³ am Tag kann einem entnommenen Substrat hinzugefügt werden, wenn die Insektenlarven noch klein sind.

20 bis 30 Liter Wasser pro m³ am Tag kann einem entnommenen Substrat hinzugefügt werden, wenn die Insektenlarven groß sind. Je größer Insektenlarven sind, umso stärker kann befeuchtet werden, um die Anzucht zu optimieren.

Zusatzstoffe wie Mineralien und/oder Aminosäuren können dem Wasser hinzugefügt sein. Wasser kann auch in Form eines Gels hinzugefügt werden.

Es können dem entnommenen Substrat Futter und/oder Mineralien und/oder Aminosäuren hinzugefügt werden, bevor das entnommene Substrat in den Behälter zurückgegeben wird. Die Anzucht kann so weiter verbessert werden.

Wird Luft durch den Zuchtbehälter geleitet, so kann dies mit einem sehr geringen Überdruck geschehen. Der Überdruck wird vorzugsweise so gering gewählt, dass Substrat nicht aufgewirbelt wird, um den Anzuchterfolg zu maximieren. Der Überdruck kann kleiner sein als 800 Millibar oder kleiner sein als 500 Millibar oder kleiner sein als 300 Millibar. Der Überdruck kann wenigstens 10 Millibar oder wenigstens 50 Millibar betragen. Luftleitelemente, die Luft durch den Zuchtbehälter hindurchleiten, sind vorzugsweise nicht länger als 3 m oder nicht länger als 4 m, damit der Überdruck sehr gering sein kann.

Je größer Insektenlarven sind, um so stärker kann der Luftstrom sein, um das Anzüchten zu optimieren.

Wird Luft durch den Zuchtbehälter geleitet, so geschieht dies während der Aufzucht vorzugsweise unterbrechungsfrei, um kontinuierlich beispielsweise Gase aus dem Zuchtbehälter zu entfernen.

Wird Substrat in den Zuchtbehälter zurückgebracht, so geschieht diese vorzugsweise mithilfe eines rotierenden Elements wie zum Beispiel einem gebogenen Rohr, das sehr langsam dreht. Die Rotationsgeschwindigkeit kann beispielsweise geringer sein als eine Umdrehung innerhalb von 15 Sekunden oder innerhalb von 30 Sekunden. Die Rotationsgeschwindigkeit kann beispielsweise größer sein als eine Umdrehung innerhalb von 5 Minuten oder innerhalb von 2 Minuten.

Die Erfindung ermöglicht es, wenigstens eine Million Insektenlarven pro Kubikmeter anzuzüchten. Es können auch wenigstens zwei Millionen Insektenlarven pro Kubikmeter sein.

Das Verhältnis von Futter zu Insektenlarven kann anfänglich so gewählt sein, dass das Futter bis zur vollständigen Aufzucht reicht. Nach einer vollständigen Aufzucht kann das Substrat zu 40% bis 50% aus Insektenlarven bestehen. Der Rest des Substrats kann aus Kot und Futterresten bestehen, wie zum Beispiel zu 50% aus Kot und zu 10% aus Futterresten. Diese Futterreste können herausgesiebt und wiederverwendet werden.

Es zeigen:
- Figur 1:: Zuchtvorrichtung;
- Figur 2:: aus Behältermodulen gebildeter Zuchtbehälter;
- Figur 3:: Behältermodulvorderseite;
- Figur 4:: Aufsicht auf Behältermodul;
- Figur 5:: Querschnitt durch Behältermodul;
- Figur 6:: Querschnitt durch Behältermodul mit Substrat;
- Figur 7:: Behältermodul;
- Figur 8:: Zuchtbehälter mit Entnahmeeinrichtung;
- Figur 9:: Rinne einer Entnahmeeinrichtung;
- Figur 10:: Rinnen einer Entnahmeeinrichtung;
- Figur 11:: Pendelbecherwerk;
- Figur 12:: Verteilereinrichtung;
- Figur 13:: Detailansicht auf Innenseite einer Zwischenwand

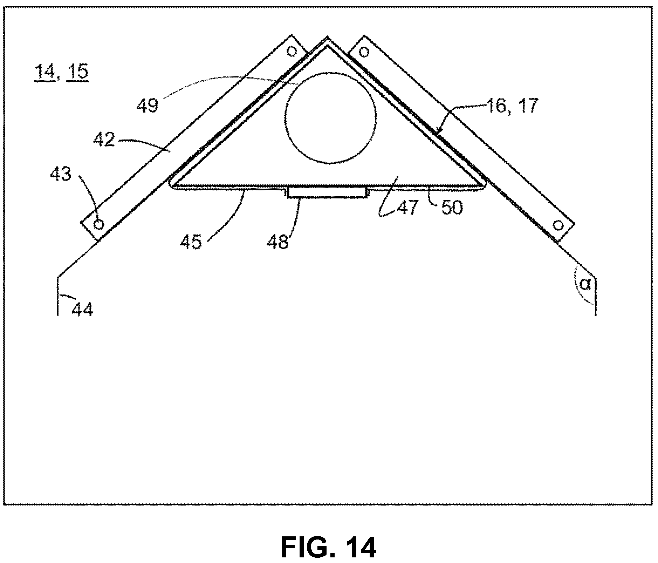

- Figur 14:: Detailansicht auf Innenseite der Zwischenwand mit eingesetztem Rohr,
- Figur 15:: Detailansicht auf Außenseite der Zwischenwand mit eingesetztem Rohr.

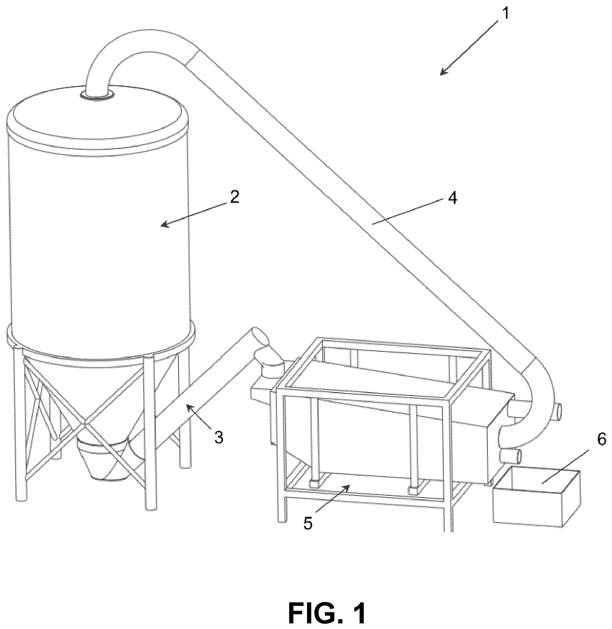

Die Figur 1 verdeutlicht ein Grundprinzip. Gezeigt wird eine Zuchtvorrichtung 1 zur Anzucht von Insektenlarven mit einem siloförmigen Zuchtbehälter 2, einer Rücktransporteinrichtung 3, 4 und einer Trenneinrichtung 5.

Im Zuchtbehälter 2 befindet sich ein feuchtes Substrat. Das Substrat umfasst Insektenlarven wie zum Beispiel Mehrwürmer und Futter für die Insektenlarven wie zum Beispiel ein Mühlennachprodukt. Ein Mühlennachprodukt ist ein Produkt, das bei der Herstellung von Mehl anfällt. Beispiele für Mühlennachprodukte sind Kleie, Bollmehl, Nachmehl und Futtermehl.

Der Zuchtbehälter 2 weist einen beispielsweise trichterförmigen unteren Bereich mit einer Substrataustrittsöffnung auf. Über die Substrataustrittsöffnung gelangt Substrat in die Rücktransporteinrichtung 3, 4 hinein. Die Rücktransporteinrichtung 3, 4 kann entnommenes Substrat beispielsweise durch Förderbänder, Becherwerk, Pendelbecherwerk und/oder Schnecken zur Oberseite des Zuchtbehälters 2 transportieren. Entnommenes Substrat kann anschließend durch eine Substrateintrittsöffnung zurück in den Zuchtbehälter 2 gegeben werden.

Haben die Insektenlarven eine gewünschte Größe erreicht, so werden diese durch die Trenneinrichtung 5 dem Substrat entnommen und beispielsweise in einen Transportbehälter 6 gegeben.

Der Zuchtbehälter 2 kann wie gezeigt auf einem Gestell abgestellt sein, damit die Unterseite des Zuchtbehälters 2 erreichbar ist.

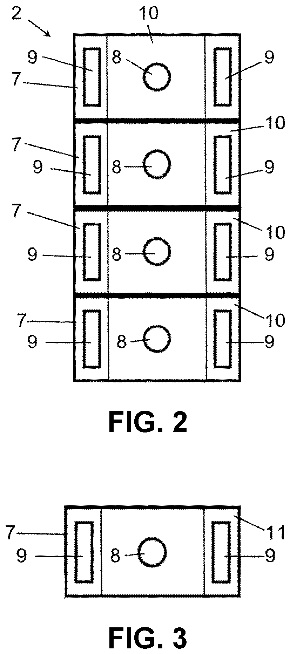

Die Figur 2 zeigt einen Zuchtbehälter 2, der aus einer Mehrzahl von Behältermodulen 7 gebildet ist. Ein jedes Behältermodul 7 ist getrennt von den anderen Behältermodulen 7 gefertigt worden. Nach der Fertigung wurden die Behältermodule 7 übereinandergestapelt. Aus Sicherheitsgründen können die übereinandergestapelten Behältermodule 7 untereinander befestigt worden sein.

Ein jedes Behältermodul 7 kann, wie in der Figur 2 gezeigt, eine Lufteintrittsöffnung 8 und ein oder zwei Luftaustrittsöffnungen 9 umfassen. Über eine jede Lufteintrittsöffnung 8 kann Luft in den Behälter 2 eingeleitet werden. Über eine jede Luftaustrittsöffnung 9 kann Luft aus dem Behälter 2 herausströmen. Gezeigt werden die Behältermodule 7 von einer Vorderseite, die durch Seitenwände 10 der Behältermodule 7 gebildet wird.

Die Figur 3 zeigt ein einzelnes Behältermodul 7 von der Rückseite mit der Seitenwand 11 und damit von der Seite, die der Seitenwand 10 gegenüberliegt. Die Figur 3 verdeutlicht, dass die vordere Seitenwand 10 und die hintere Seitenwand 11 gleich sein können.

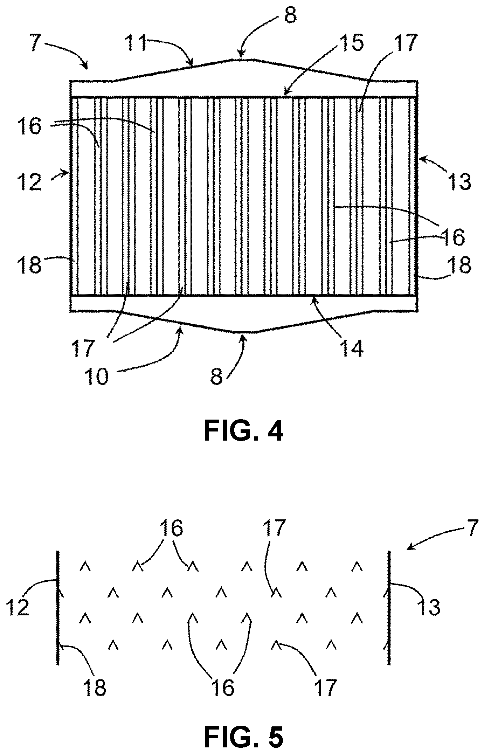

Die Figur 4 zeigt eine Aufsicht auf die obere Seite eines Behältermoduls 7. Das Behältermodul 7 weist die beiden Seitenwände 10 und 11 sowie daran angrenzende Seitenwände 12 und 13 auf. Es gibt zwei Zwischenwände 14 und 15. Die Seitenwand 11 schließt mit der Zwischenwand 15 einen Raum ein. Die Seitenwand 10 schließt mit der Zwischenwand 14 einen Raum ein. Im Inneren des Behältermoduls 7 gibt es Luftleitelemente 16 und Luftleitelemente 17. Die Luftleitelemente 16 sind in einer obersten Ebene angeordnet. Die Luftleitelemente 17 sind in einer darunter liegenden Ebene angeordnet. Es gibt sechs Luftleitelemente 16 und fünf Luftleitelemente 17. Von oben gesehen ist ein jedes Luftleitelement 17 zwischen zwei Luftleitelemente 16 angeordnet.

Bei den Seitenwänden 12 und 13 können Schrägen 18 angeordnet sein, die aber keine Luftleitelemente sein müssen. Stattdessen können diese Schrägen 18 nur dazu dienen, ein Substrat vor übermäßig großen Druck zu schützen. Schrägen 18 können auf Höhe der Luftleitelemete 17 angeordnet sein.

Unter der Ebene mit den Luftleitelementen 17 kann sich eine weitere Ebene mit weiteren Luftleitelementen 16 befinden. Unter der weiteren Ebene mit den weiteren Luftleitelementen 16 kann sich eine weitere Ebene mit weiteren Luftleitelementen 17 befinden und so weiter.

Luft kann durch die Luft - Eintrittsöffnung 8 der Seitenwand 11 durch die Luftleitelemente 16 hindurch zu den Luft - Austrittsöffnungen der Seitenwand 10 strömen. Luft kann außerdem durch die Luft - Eintrittsöffnung 8 der Seitenwand 10 durch die Luftleitelemente 17 hindurch zu den Luft - Austrittsöffnungen der Seitenwand 11 strömen.

In der Figur 5 wird ein Querschnitt durch ein Behältermodul 7 gezeigt. Die Luftleitelemente 16 und 17 sind in unterschiedlichen, horizontalen Ebenen angeordnet. Es gibt eine erste Ebene mit beispielsweise sechs Luftleitelementen 16. Es kann dann eine dritte Ebene mit sechs Luftleitelementen 16 geben. Zwischen der ersten und der dritten Ebene gibt es eine Ebene mit beispielsweise fünf Luftleitelementen 17. Unterhalb der dritten Ebene gibt eine Ebene mit beispielsweise fünf Luftleitelementen 17. Die Ebenen weisen gleiche Abstände auf. Die Luftleitelemente 17 sind versetzt zu den Luftleitelementen 16 angeordnet. An den Seitenwänden können beispielsweise auf Höhe der zweiten und vierten Ebene Schrägen 18 befestigt sein, die darunter befindliches Substrat vor einem übermäßig großen Druck schützen können. Die Schräge 17 sind wie die Luftleitelemente 16 und 17 nach unten offen. Der Querschnitt der Luftleitelemente 16 und 17 kann, wie in der Figur 5 gezeigt, die Form von einem V haben, das auf den Kopf gestellt ist.

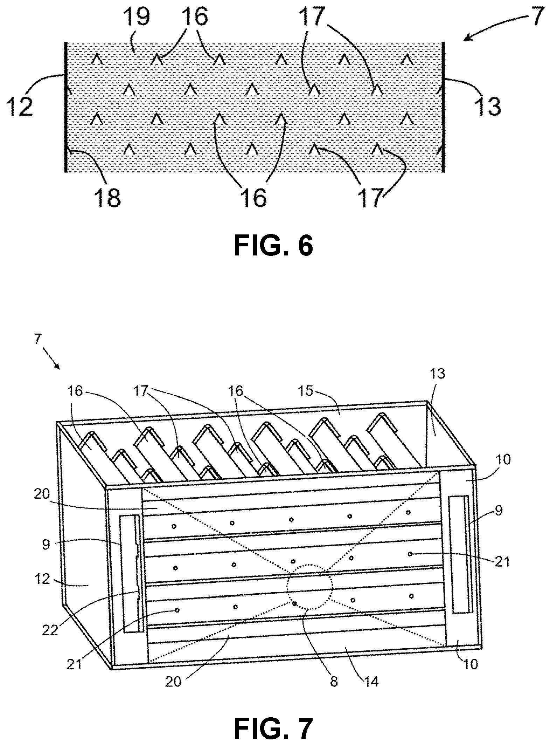

In der Figur 6 wird das Behältermodul 7 aus Figur 5 gezeigt, das nun mit Substrat 19 von oben gefüllt worden ist. Da die Luftleitelemente 16 und 17 nur nach unten offen sind, füllen diese sich zumindest nicht sofort mit Substrat. Durch den Querschnitt der Luftleitelemente 16 und 17 kann daher Luft hindurchströmen. Dies gilt auch für die Schrägen 18, die daher bei Bedarf auch Luftleitelemente sein können.

Die Figur 7 zeigt ein Behältermodul 7, bei der die vordere Seitenwand 10 teilweise transparent dargestellt ist. Die Zwischenwand 14 ist daher teilweise sichtbar. An der Zwischenwand 14 sind Abluftkanäle 20 angebracht, in die die Luft über Löcher in der Zwischenwand hineinströmt, die von den Luftleitelementen 16 kommt. Zwischen zwei Abluftkanälen 20 gibt es jeweils eine Reihe mit jeweils fünf Löchern 21. In die Löcher 21 strömt die Luft hinein, die zuvor durch die Lufteintrittsöffnung 8 der Seitenwand 10 hineingeströmt ist. Hat die Luft die Löcher 21 passiert, so gelangt die Luft in als Kanäle ausgebildete Luftleitelemente 17 hinein. Luft strömt dann die Luftleitelemente 17 entlang, um dann über Löcher in Abluftkanäle zu gelangen, die an der Außenseite der hinteren Zwischenwand 15 in Höhe der Luftleitelemente 17 angebracht sind und ebenfalls wie die Abluftkanäle 20 horizontal verlaufen.

Die offenen Enden 22 der Abluftkanäle 20 enden bei den Luftaustrittsöffnungen 9, über die Luft dann aus dem Zuchtbehälter 7 austritt.

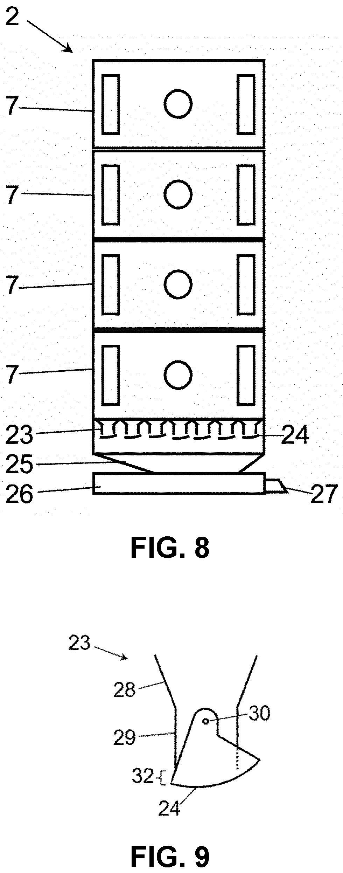

Die Figur 8 zeigt einen Zuchtbehälter 2 mit einer Entnahmeeinrichtung. Die Entnahmeeinrichtung ist an der Unterseite des untersten Behältermoduls 7 angebracht. Die Entnahmeeinrichtung kann eine Mehrzahl an Stutzen oder Rinnen 23 umfassen, um Substrat schonend entnehmen zu können. Stutzen oder Rinnen 23 können an ihrer Unterseite eine Abdeckung 24 aufweisen, durch die eine Öffnung an der Unterseite des Stutzens oder der Rinne geöffnet sowie geschlossen werden kann. Ein Stutzen oder eine Rinne 23 können in Aufsicht gesehen so zwischen zwei Luftleitelementen der untersten Ebene angeordnet sein, dass Substrat durch zwei Luftleitelemente in einen dazwischenliegenden Stutzen oder eine dazwischenliegende Rinne 23 gelenkt wird. Die Entnahmeeinrichtung kann unterhalb der Rinnen 23 einen Auffangbehälter 25 umfassen, in den Substrat hineinfallen kann. Der Auffangbehälter 25 kann ein oder mehrere schräge Wände aufweisen, um Substrat beispielsweise zu einer Fördereinrichtung 26 zu lenken. Die Fördereinrichtung 26 kann einen Auslass 27 aufweisen, über den Substrat die Entnahmeeinrichtung verlassen kann. Über den Auslass 27 kann Substrat beispielsweise in Becher eines Becherwerks gelangen. Die Becher können sich über eine Länge von wenigstens einem Meter oder wenigstens zwei Metern erstrecken. Die Länge kann weniger als 5 m oder weniger 4 m oder weniger als 3 m betragen. Die Fördereinrichtung 26 kann einen Schieber oder ein anders Transportmittel umfassen, um Substrat zum Auslass 27 schieben zu können. Die Fördereinrichtung 26 kann alternativ durch eine langsame Bewegung in Richtung Auslass 27 und durch eine schnelle Rückbewegung sehr schonend in Richtung Auslass gefördert werden. Die Fördereinrichtung 26 kann ein Muldenförderband für den Transport umfassen. Die Fördereinrichtung 26 kann alternativ das Substrat durch Vibration fördern. Zum Beispiel durch eine Vibrationsrinne.

Der Auffangbehälter 25 kann an seiner Unterseite durch eine Klappe oder durch einen Schieber geschlossen sein. Um Substrat zu entnehmen, kann erst die Unterseite des Auffangbehälters geöffnet werden, um eine Restentleerung des Auffangbehälters 25 zu erreichen. Im Anschluss daran können die Abdeckungen 24 in die geöffnete Stellung bewegt werden. Soll die Entnahme beendet werden, so können zuerst die Abdeckungen 24 in die geschlossene Stellung bewegt werden. Danach kann die Unterseite des Auffangbehälters 25 geschlossen werden. Es wird so sichergestellt, dass Substrat, welches nach dem Schließen eine Abdeckung beispielsweise aufgrund eines vorhandenen Abstandes passieren kann, in denselben Zuchtbehälter 2 zurücktransportiert wird.

Die Figur 9 zeigt eine Rinne 23 im Querschnitt. Die Rinne 23 kann sich im Querschnitt zunächst verjüngen, also Schrägen 28 aufweisen, um Substrat geeignet zu lenken. Im Anschluss daran kann eine jede Rinne 23 sich senkrecht nach unten erstrecken und also senkrecht nach unten verlaufende Seitenwände 29 aufweisen. Die Rinne 23 kann an der Oberseite zum Zuchtbehälter 2 hin dauerhaft geöffnet sein. Die Rinne 23 kann an der Oberseite zum Zuchtbehälter 2 hin aber auch verschließbar sein, so zum Beispiel mithilfe einer oberen Abdeckung, die für ein Öffnen und Schließen in einer Ebene verschoben werden kann. Die Abdeckung 24 an der Unterseite der Rinne 23 kann bogenförmig verlaufen und um eine Achse 30 für ein Öffnen bzw. Schließen verschwenkt werden, um besonders substratschonend öffnen und schließen zu können. Die Abdeckung 24 kann einen Abstand 32 von zum Beispiel wenigstens 1 cm und/oder maximal 4 cm aufweisen, um besonders substratschonend öffnen und schließen zu können. Die Rinne 23 kann sich über die gesamte Tiefe des Zuchtbehälters 2 erstrecken, um über die gesamte Tiefe Substrat vollständig entnehmen zu können.

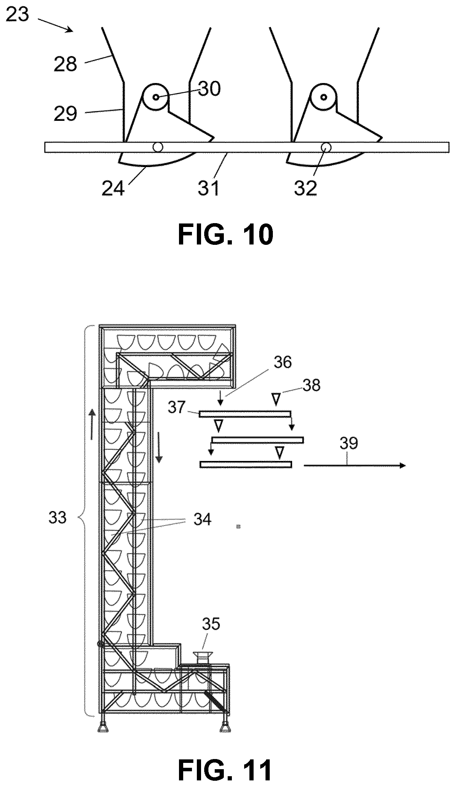

Um mit nur einem Antrieb sämtliche Abdeckungen 24 gemeinsam öffnen und schließen zu können, können die Abdeckungen untereinander durch eine in der Figur 10 gezeigte Stange 31 miteinander verbunden sein. Beispielsweise über einen Bolzen 32 oder eine Schraube kann eine jede Abdeckung 23 drehbeweglich mit der Stange 31 verbunden sein. Wird die in der Figur 10 gezeigte Stange 31 durch einen Antrieb nach rechts bewegt, so werden sämtliche Abdeckungen 24 geöffnet. Durch eine Bewegung der Stange 31 in die entgegengesetzte Richtung werden die Abdeckungen 24 wieder geschlossen.

In der Figur 11 wird ein Pendelbecherwerk 33 gezeigt, mit dem Substrat von unten nach oben transportiert werden kann. Das Pendelbecherwerk 33 kann also Teil der Rücktransporteinrichtung sein, um entnommenes Substrat zurück in einen Zuchtbehälter zu transportieren. Das Pendelbecherwerk 33 umfasst eine Vielzahl von Bechern 34. Über einen Einlass 35 gelangt Substrat in einen Becher 34 hinein und wird nach oben zu einem Auslass 36 transportiert. Der Einlass 35 kann ein Trichter oder ein Dosiersytem sein, von dem aus Substrat in einen Becher 34 hineinfallen kann. Der Auslass 36 kann dadurch realisiert sein, dass ein Becher 34 beim Auslass so gedreht wird, dass seine Öffnung nach unten weist. Substrat kann dann aus dem Becher 34 herausfallen. Substrat kann vom Auslass 36 auf eine oberste Platte 37 fallen. Die Platte 37 kann langsam in Richtung Düse 38 bewegt werden und schnell zurück, um so Substrat besonders schonend zur Düse 38 zu bewegen. Die Platte 37 kann alternativ das Substrat durch Vibration fördern. Zum Beispiel durch eine Vibrationsrinne. Die Düse 38 befindet sich an dem von dem Auslass 36 entfernten Ende der Platte 37. Durch die Düse 38 wird das Substrat beispielsweise mit Wasser befeuchtet und zwar vorzugsweise nur ein Oberflächenbereich des Substrats. Das Substrat wird weiter nach rechts transportiert und fällt schließlich auf eine darunter befindliche Platte 17, wie durch einen Pfeil angedeutet. Substrat wird dann zur darunter befindlichen Düse transportiert, die sich in Bewegungsrichtung des Substrats gesehen wieder beim Ende der zweiten Platte befindet. So gelangt Substrat schließlich homogen und schonend befeuchtet zur Fördereinrichtung 39. Die Fördereinrichtung 39 transportiert das Substrat zur Substrateintrittsöffnung des gewünschten Zuchtbehälters. Die Fördereinrichtung 39 kann ein Muldenförderband sein oder umfassen, um Substrat schonend zu transportieren. Ein Muldenförderband kann ein Förderband sein, dessen Oberfläche beispielsweise Rippen aufweisen kann, um Mulden zu bilden. In die Mulden gelangt Substrat hinein und wird so gefördert. Ein Muldenförderband kann keine ebene Oberfläche aufweisen, sondern erhöhte seitliche Ränder. Ein Muldenförderband kann im Schnitt V-förmig oder U-förmig sein.

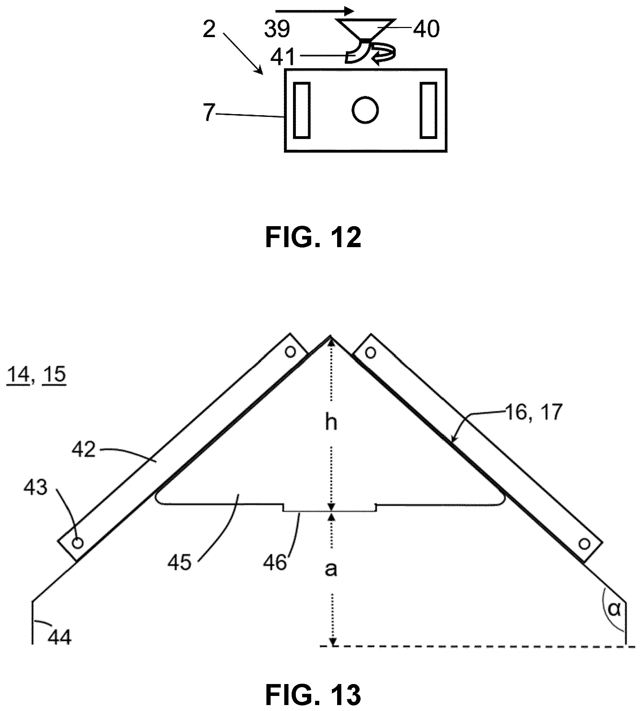

In der Figur 12 wird eine Verteilereinrichtung gezeigt. Substrat kann über die Fördereinrichtung 39 zur Verteilereinrichtung gebracht werden. Die Verteilereinrichtung kann einen Trichter 40 umfassen, in den hinein das Substrat gebracht werden kann. Die Verteilereinrichtung kann ein drehbares Rohr 41 umfassen. Das Rohr 41 kann so bogenförmig verlaufen, dass Substrat ringförmig verteilt in den Zuchtbehälter 2 hineinfallen kann, wenn sich das Rohr 41 wie durch einen Pfeil angedeutet dreht.

Die Figur 13 zeigt eine Detailansicht einer Zwischenwand 14, 15 von innen gesehen. Dargestellt ist ein Bereich der Zwischenwand 14, 15 mit einem Luftleitelement 16, 17. Das Luftleitelement 16, 17 kann aus einem Blechzuschnitt und einem anschließenden Umformen hergestellt worden sein. Das Luftleitelement 16, 17 kann an beiden Enden umgebogene Laschen 42 aufweisen. In die Laschen 42 können ein oder mehrere Löcher hineingebohrt worden sein, um das Luftleitelement 16, 17 an der Zwischenwand 14, 15 beispielsweise durch Nieten oder eine Schraubverbindung 43 zu befestigen.

Um die Druckfestigkeit des Luftleitelements 16, 17 vorteilhaft zu erhöhen, kann das Luftleitelement 16, 17 zunächst wie ein um 180° gedrehtes V bzw. wie ein Giebeldach im Querschnitt geformt sein. An die beiden Schenkel der V-Form bzw. der Giebeldachform können sich dann im eingebauten Zustand senkrecht verlaufende Abschnitte 44 anschließen, um die Druckfestigkeit des Luftleitelements 16, 17 zu verbessern. Ein Abschnitt 44 kann daher mit einem Schenkel einen stumpfen Winkel α von mehr als 70° und weniger als 180° einschließen.

In der Zwischenwand 14, 15 kann es für ein jedes Luftleitelement 16, 17 eine Wandöffnung 45 geben. Die Wandöffnung 45 kann im Wesentlichen wie ein Dreieck geformt sein. Zum Beispiel an der Unterseite der Wandöffnung 45 kann es eine Aussparung 46 geben. Die Aussparung 46 kann mittig bei der Unterseite angeordnet sein. Die Höhe h der Wandöffnung 45 kann, wie in der Figur 13 gezeigt, kleiner als die Höhe des Luftleitelements 16, 17 sein. Die Wandöffnung 45 kann an die Oberseite des Luftleitelements 16, 17 angrenzen, so dass die Unterseite der Wandöffnung 45 einen Abstand a von der Unterseite des Luftleitelements 16, 17 aufweist. Der Abstand a kann beispielsweise ähnlich groß wie die Höhe h der Wandöffnung 45 sein. Der Abstand a kann wenigstens halb so groß sein wie die Höhe h der Wandöffnung 45.

Durch den Abstand a kann vorteilhaft erreicht werden, dass kein Substrat unerwünscht in die Lufteintrittsöffnung bzw. die Luftaustrittsöffnung gelangen kann.

In die Wandöffnung 45 kann von außen ein Rohr 47 hineingesteckt werden. Das Rohr 47 kann ein Rastelement 48 aufweisen. Ist das Rohr 47 in die Wandöffnung 45 hineingesteckt worden, so rastet das Rastelement 48 auf der Innenseite der Zwischenwand 14, 15 ein und kann dann an der Innenseite der Zwischenwand 14, 15 anliegen. Das Rohr 47 kann dann formschlüssig gehalten sein. Das Rohr 47 mit dem Rastelement 48 kann einteilig in einem Arbeitsschritt zum Beispiel aus Kunststoff hergestellt worden sein, so zum Beispiel durch Spritzgießen. Das Rohr 47 und das Rastelement 48 können aus Metall bestehen. Das Rastelement 48 kann getrennt von dem Rohr hergestellt worden sein. Das Rastelelement kann eine Feder und einen Schieber umfassen. Der Schieber kann beispielsweise gegen die Kraft der Feder in eine geöffnete Stellung und damit aus einer Raststellung heraus verschoben werden.

Das Rohr 47 kann einen Abschnitt mit einem ersten, zum Beispiel kreisrunden Querschnitt 49 aufweisen, an den sich ein Abschnitt mit einem zweiten, zum Beispiel dreiecksförmigen Querschnitt 50 anschließen kann. Die Querschnittsfläche des ersten Querschnitts 49 kann kleiner als die Querschnittsfläche des zweiten Querschnitts 50 sein. Dies kann dazu beitragen, dass Substrat vorteilhaft nicht durch einen Luftstrom aufgewirbelt wird. Die Form des zweiten Querschnitts 50 kann an die Form der Wandöffnung 45 angepasst sein. Beide Formen können also zumindest im Wesentlichen wie ein Dreieck geformt sein. Dies ist insbesondere dann der Fall, wenn das Luftleitelement 16, 17 wie ein umgedrehtes V geformt ist. Ist das Luftleitelement 16, 17 wie ein umgedrehtes U geformt, dann ist zu bevorzugen, dass beide Formen an die umgedrehte U-Form angepasst sind.

Das Rohr 47 bildet dann die Lufteintrittsöffnung bzw. die Luftaustrittsöffnung.

Die Aussparung 46 kann dafür vorgesehen sein, damit das Rastelement 48 zusammen mit dem zweiten Querschnitt 50 durch die Wandöffnung 45 geeignet hindurchgedrückt werden kann. Das Rastelement 46 kann so sein, dass dieses zusammen mit dem Rohr 47 zerstörungsfrei wieder herausgezogen werden kann, so zum Beispiel zu Wartungszwecken oder Reparaturzwecken.

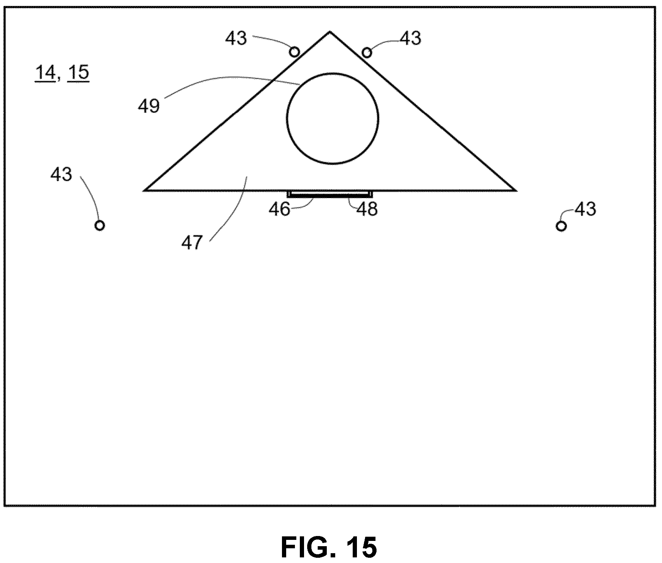

Die Figur 15 zeigt die Aufsicht auf die Vorderseite der Zwischenwand 14, 15. Das Rohr 47 ist in die Wandöffnung 45 eingesetzt worden. Der Übergang 51 des Rohrs 47 zwischen dem ersten Abschnitt 49 und dem zweiten Abschnitt 50 kann zumindest stellenweise oder, wie in der Figur 15 gezeigt, vollständig etwas größer als die Wandöffnung 45 sein, damit das Rohr 47 formschlüssig gehalten werden kann. Daher ist in der Figur 15 von der Wandöffnung 45 nur noch die Aussparung 46 zu erkennen.

Der erste Abschnitt 50 des Rohrs 47 kann an seiner Außenseite gewellt sein oder anders geformte umlaufende Rippen aufweisen, um den ersten Abschnitt 50 mit einem Schlauch zuverlässig verbinden zu können.

Eine Zwischenwand 14, 15 ist nicht zwingend erforderlich. Eine Zwischenwänd 14, 15 kann beispielsweise dann entfallen, wenn Lufteintrittsöffnungen und/oder Luftaustrittsöffnungen unmittelbar zum Beispiel mit Schläuchen verbunden werden. Eine Seitenwand 10, 11 kann dann so beschaffen sein, wie dies zuvor für eine Zwischenwand 14, 15 beschrieben wurde.

## Patentansprüche

1. Zuchtvorrichtung für eine Aufzucht von Insektenlarven, mit einem Zuchtbehälter (2) zur Aufnahme von Substrat (19) und mit einer Befeuchtungseinrichtung (37, 38), mit dem Substrat (19) befeuchtet werden kann, **gekennzeichnet durch** eine Entnahmeeinrichtung (23 bis 27), mit der Substrat (19) aus dem Zuchtbehälter (2) entnommen werden kann, wobei die Befeuchtungseinrichtung (37, 38) so eingerichtet ist, dass diese das durch die Entnahmeeinrichtung (23 bis 27) entnommene Substrat (19) befeuchten kann.

2. Zuchtvorrichtung nach dem vorhergehenden Anspruch, mit einer Rücktransporteinrichtung (3, 4), mit der Substrat (19), das durch die Entnahmeeinrichtung (23 bis 27) entnommen wird, zurück in den Zuchtbehälter (2) transportiert werden kann.

3. Zuchtvorrichtung nach einem der vorhergehenden Ansprüche, mit einer Verdunstungseinrichtung (16, 17) für ein Kühlen, mit der Flüssigkeit im Zuchtbehälter (2) verdunstet werden kann.

4. Zuchtvorrichtung nach einem der vorhergehenden Ansprüche, **dadurch gekennzeichnet, dass** mehrere Zuchtbehälter (2) vorhanden sind.

5. Zuchtvorrichtung nach dem vorhergehenden Anspruch, **dadurch gekennzeichnet, dass** es für jeden Zuchtbehälter (2) eine eigene Entnahmeeinrichtung (23 bis 27) gibt und zumindest ein Teil (33) einer Rücktransportvorrichtung (3, 4) einen Rücktransport von entnommenen Substrat (19) zu den Zuchtbehältern (2) bewirkt.

6. Zuchtvorrichtung nach einem der vorhergehenden Ansprüche, **dadurch gekennzeichnet, dass** ein oder mehrere Luftleitelemente (16, 17) in einem Zuchtbehälter (2) vorhanden sind, über die Luft durch den Zuchtbehälter (2) hindurchströmen kann.

7. Zuchtvorrichtung nach dem vorhergehenden Anspruch, **dadurch gekennzeichnet, dass** ein jedes Luftleitelement (16, 17) eine offene Unterseite aufweist.

8. Zuchtvorrichtung nach einem der beiden vorhergehenden Ansprüche, **dadurch gekennzeichnet, dass** die Oberseite eines jeden Luftleitelements (16, 17) durch eine Kante gebildet wird und sich ein jedes Luftleitelement (16, 17) nach unten hin verbreitert.

9. Zuchtvorrichtung nach einem der drei vorhergehenden Ansprüche, **dadurch gekennzeichnet, dass** Luftleitelemente (16) in einer ersten Ebene in dem Zuchtbehälter (2) angeordnet sind und Luftleitelemente (17) in einer zweiten Ebene in dem Zuchtbehälter (2) angeordnet sind, wobei die Luftleitelemente (16) der einen Ebene versetzt zu den Luftleitelementen (17) der anderen Ebene angeordnet sind.

10. Zuchtvorrichtung nach einem der vorhergehenden Ansprüche, **dadurch gekennzeichnet, dass** ein Gebläse zur Erzeugung eines Luftstroms vorhanden ist, der durch einen Zuchtbehälter (2) hindurchgeleitet wird.

11. Zuchtvorrichtung nach einem der vorhergehenden Ansprüche, **dadurch gekennzeichnet, dass** der Zuchtbehälter (2) zwei Lufteintrittsöffnungen (8) an gegenüberliegenden Seiten aufweist und zwei Luftaustrittsöffnungen (9) an den gegenüberliegenden Seiten aufweist, wobei die Zuchtvorrichtung so eingerichtet ist, dass Luft von einer jeden Lufteintrittsöffnung (8) zur Luftaustrittsöffnung (9) der gegenüberliegenden Seite geleitet wird.

12. Zuchtvorrichtung nach einem der vorhergehenden Ansprüche, wobei die Befeuchtungseinrichtung (37, 38) so eingerichtet ist, dass entnommenes Substrat (19) schrittweise befeuchtet wird.

13. Zuchtvorrichtung nach dem vorhergehenden Anspruch, **dadurch gekennzeichnet, dass** die Befeuchtungseinrichtung (37, 38) so eingerichtet ist, dass zwischen zwei Befeuchtungsschritten entnommenes Substrat (19) gemischt und/oder umgewälzt wird.

14. Zuchtvorrichtung nach einem der vorhergehenden Ansprüche, mit einer Steuereinrichtung, mit der die Flüssigkeitszufuhr zu entnommenem Substrat (19) und/oder die Luftzufuhr zum Zuchtbehälter (2) gesteuert wird, wobei die Steuereinrichtung so eingerichtet ist, dass die Flüssigkeitszufuhr zu entnommenem Substrat (19) und/oder Luftzufuhr in den Zuchtbehälter (2) hinein in Abhängigkeit von der Größe von Insektenlarven gesteuert wird.

15. Verfahren für ein Anzüchten von Insektenlarven mit einer Zuchtvorrichtung nach einem der vorhergehenden Ansprüche mit den Schritten:
Substrat (19) umfassend Insektenlarven und Futter für die Insektenlarven wird in einen Zuchtbehälter (2) der Zuchtvorrichtung gegeben;
innerhalb von 12 Stunden bis fünf Tagen wird das Substrat (19) von der Unterseite des Zuchtbehälters (2) vollständig oder zu wenigstens 80% oder zu wenigstens 90% entnommen und über die Oberseite des Zuchtbehälters (2) wieder in den Zuchtbehälter (2) gebracht.

## Claims

1. Breeding apparatus for breeding insect larvae, having a breeding container (2) for receiving substrate (19) and having a humidifying device (37, 38) by means of which substrate (19) can be humidified, **characterised by** an extraction device (23 to 27), by means of which substrate (19) can be extracted from the breeding container (2), wherein the humidifying device (37, 38) is configured such that it can humidify the substrate (19) extracted by the extraction device (23 to 27).

2. Breeding apparatus according to the preceding claim, having a return transport device (3, 4), by means of which substrate (19), extracted by the extraction device (23 to 27), can be transported back into the breeding container (2).

3. Breeding apparatus according to any one of the preceding claims, having an evaporation device (16, 17) for cooling, by means of which liquid in the breeding container (2) can be evaporated.

4. Breeding apparatus according to any one of the preceding claims, **characterised in that** a plurality of breeding containers (2) are provided.

5. Breeding apparatus according to the preceding claim, **characterised in that** there is a separate extraction device (23 to 27) for each breeding container (2) and at least one part (33) of a return transport device (3, 4) causes a return transport of extracted substrate (19) to the breeding containers (2).

6. Breeding apparatus according to any one of the preceding claims, **characterised in that** one or more air-guiding elements (16, 17) are provided in a breeding container (2), through which air can flow through the breeding container (2).

7. Breeding apparatus according to the preceding claim, **characterised in that** each air-guiding element (16, 17) has an open underside.

8. Breeding apparatus according to any one of the two preceding claims, **characterised in that** the upper side of each air-guiding element (16, 17) is formed by an edge and each air-guiding element (16, 17) widens towards the bottom.

9. Breeding apparatus according to any one of the three preceding claims, **characterised in that** air-guiding elements (16) are arranged in a first plane in the breeding container (2) and air-guiding elements (17) are arranged in a second plane in the breeding container (2), wherein the air-guiding elements (16) of one plane are arranged offset to the air-guiding elements (17) of the other plane.

10. Breeding apparatus according to any one of the preceding claims, **characterised in that** there is a fan for generating an air flow which is directed through a breeding container (2).

11. Breeding apparatus according to any one of the preceding claims, **characterised in that** the breeding container (2) has two air inlet openings (8) on opposite sides and has two air outlet openings (9) on the opposite sides, wherein the breeding apparatus is configured such that air is directed from each air inlet opening (8) to the air outlet opening (9) of the opposite side.

12. Breeding apparatus according to any one of the preceding claims, wherein the humidifying device (37, 38) is configured so that extracted substrate (19) is humidified in steps.

13. Breeding apparatus according to the preceding claim, **characterised in that** the humidifying device (37, 38) is configured so that substrate (19) extracted between two humidifying steps is mixed and/or circulated.

14. Breeding apparatus according to any one of the preceding claims, having a control device for controlling the supply of liquid to extracted substrate (19) and/or the supply of air to the breeding container (2), wherein
the control device is configured such that the supply of liquid to the extracted substrate (19) and/or supply of air to the breeding container (2) is controlled depending on the size of insect larvae.

15. Method for breeding insect larvae with a breeding apparatus according to any one of the preceding claims comprising the following steps:
substrate (19) comprising insect larvae and feed for the insect larvae is placed in a breeding container (2) of the breeding apparatus;
within 12 hours to five days, the substrate (19) is extracted completely or to at least 80% or at least 90% from the underside of the breeding container (2), and returned into the breeding container (2) via the upper side of the breeding container (2).

## Revendications

1. Dispositif d'élevage pour l'élevage de larves d'insectes, comportant un récipient d'élevage (2) pour recevoir du substrat (19) et comportant un système d'humidification (37, 38) permettant d'humidifier le substrat (19), **caractérisé par** un système de prélèvement (23 à 27) permettant de prélever le substrat (19) du récipient d'élevage (2), dans lequel le système d'humidification (37, 38) est configuré de telle sorte que celui-ci peut humidifier le substrat (19) prélevé par le système de prélèvement (23 à 27).

2. Dispositif d'élevage selon la revendication précédente, comportant un système de transport de retour (3, 4) permettant de transporter en retour le substrat (19) prélevé par le système de prélèvement (23 à 27) dans le récipient d'élevage (2).

3. Dispositif d'élevage selon l'une quelconque des revendications précédentes, comportant un système de refroidissement par évaporation (16, 17), permettant d'évaporer du liquide dans le récipient d'élevage (2).

4. Dispositif d'élevage selon l'une quelconque des revendications précédentes, **caractérisé en ce que** plusieurs récipients d'élevage (2) sont présents.

5. Dispositif d'élevage selon la revendication précédente, **caractérisé en ce qu'**il existe pour chaque récipient d'élevage (2) un système de prélèvement (23 à 27) propre et au moins une partie (33) d'un dispositif de transport de retour (3, 4) entraîne un transport de retour de substrat (19) prélevé vers les récipients d'élevage (2).

6. Dispositif d'élevage selon l'une quelconque des revendications précédentes, **caractérisé en ce qu'**un ou plusieurs éléments de guidage d'air (16, 17) sont présents dans un récipient d'élevage (2), par lesquels de l'air peut circuler à travers le récipient d'élevage (2).

7. Dispositif d'élevage selon la revendication précédente, **caractérisé en ce que** chaque élément de guidage d'air (16, 17) présente un côté inférieur ouvert.

8. Dispositif d'élevage selon l'une quelconque des deux revendications précédentes, **caractérisé en ce que** le côté supérieur de chaque élément de guidage d'air (16, 17) est formé par un bord et que chaque élément de guidage d'air (16, 17) s'élargit vers le bas.

9. Dispositif d'élevage selon l'une quelconque des trois revendications précédentes, **caractérisé en ce que** des éléments de guidage d'air (16) sont disposés à un premier niveau dans le récipient d'élevage (2) et des éléments de guidage d'air (17) sont disposés à un deuxième niveau dans le récipient d'élevage (2), dans lequel les éléments de guidage d'air (16) d'un niveau sont disposés de manière décalée par rapport aux éléments de guidage d'air (17) de l'autre niveau.

10. Dispositif d'élevage selon l'une quelconque des revendications précédentes, **caractérisé en ce qu'**une soufflante est présente pour générer un flux d'air qui est acheminé à travers un récipient d'élevage (2).

11. Dispositif d'élevage selon l'une quelconque des revendications précédentes, **caractérisé en ce que** le récipient d'élevage (2) présente deux ouvertures d'entrée d'air (8) sur des côtés opposés et présente deux ouvertures de sortie d'air (9) sur des côtés opposés, dans lequel le dispositif d'élevage est mis au point de telle sorte que de l'air est acheminé depuis chaque ouverture d'entrée d'air (8) vers l'ouverture de sortie d'air (9) du côté opposé.

12. Dispositif d'élevage selon l'une quelconque des revendications précédentes, dans lequel le système d'humidification (37, 38) est configuré de sorte à humidifier progressivement le substrat (19) prélevé.

13. Dispositif d'élevage selon la revendication précédente, **caractérisé en ce que** le système d'humidification (37, 38) est configuré de sorte à mélanger et/ou faire circuler le substrat (19) prélevé entre deux étapes d'humidification.

14. Dispositif d'élevage selon l'une quelconque des revendications précédentes, avec un système de commande permettant de commander l'arrivée de liquide à un substrat (19) prélevé et/ou l'arrivée d'air au récipient d'élevage (2), dans lequel
le système de commande est configuré de sorte à commander l'arrivée de liquide du substrat (19) prélevé et/ou l'arrivée d'air dans le récipient d'élevage (2) en fonction de la taille de larves d'insectes.

15. Procédé d'élevage de larves d'insectes avec un dispositif d'élevage selon l'une quelconque des revendications précédentes, comprenant les étapes suivantes :
du substrat (19) comprenant des larves d'insectes et de la nourriture pour les larves d'insectes est placé dans un récipient d'élevage (2) du dispositif d'élevage ;
dans un délai de 12 heures à cinq jours, le substrat (19) est prélevé en totalité ou à au moins 80 % ou à au moins 90 % du côté inférieur du récipient d'élevage (2) et est replacé dans le récipient d'élevage (2) par le côté supérieur du récipient d'élevage (2).
